# EUROPEAN PATENT APPLICATION

(11) **EP 2 392 568 A1**
(43) Date of publication of application: **07.12.2011**
(21) Application number: 10075241.9
(22) Date of filing: 04.06.2010
(51) Int. Cl.: C07D 307/24, C07D 309/14, C07D 515/04, A61K 51/04

(54) **Heterocyclic amino acids for prostate cancer imaging**

(71) Applicant: Bayer Pharma Aktiengesellschaft, 13353 Berlin (DE)
(72) Inventor: Böhnke, Niels, Dr., 12157 Berlin (DE)

(57) **Abstract**

This invention relates to heterocyclic amino acid derivatives suitable for labeling or already labeled with ¹⁸F or 19F, methods of preparing such compounds, compositions comprising such compounds, kits comprising such compounds or compositions and uses of such compounds, compositions or kits for imaging proliferative diseases.

## Description

### Field of Invention

This invention relates to heterocyclic amino acid derivatives suitable for labeling or already labeled with ¹⁸ F or ¹⁹F, methods of preparing such compounds, compositions comprising such compounds, kits comprising such compounds or compositions and uses of such compounds, compositions or kits for imaging proliferative diseases.

### Background

The invention relates to the subject matter referred to in the claims, i.e. fluoro labelled heterocyclic amino acid derivatives of the formulas (I), (IIa) and (IIb), their precursors of the formula (IIIa) and (IIIb), their use and their preparation processes.

Molecular imaging has the potential to detect disease progression or therapeutic effectiveness earlier than most conventional methods in the fields of oncology, neurology and cardiology. Of the several promising molecular imaging technologies having been developed as optical imaging and MRI, PET is of particular interest for drug development because of its high sensitivity and ability to provide quantitative and kinetic data.
Prostate cancer is a leading cancer in the world, in particular in the US population where it is the second leading cause of cancer-related deaths in men. There are more than 300,000 new cases of prostate cancer diagnosed each year in the United States. Approximately US$2 billion is currently spent worldwide on surgical, radiation, drug therapy and minimally invasive treatments. Currently there is no effective therapy for relapsing, metastatic, castration-resistant prostate cancer. New agents that image prostate cancer are needed, preferably imaging agents containing radioisotopes, in particular positron emitting isotopes. Positron emitting isotopes include among other carbon, nitrogen, and oxygen. These isotopes can replace their non-radioactive counterparts in target compounds to produce tracers that function biologically and are chemically and biologically identical to the original molecules for PET imaging. On the other hand, ¹⁸F is the most convenient labelling isotope due to its relatively long half life (109.6 min) which permits the preparation of diagnostic tracers and subsequent study of biochemical processes. In addition, its high ß+ yield and low ß+ energy (635 keV) are also advantageous.
The best known example for PET imaging of diseases is 2-[18F]fluorodeoxyglucose ([18F]FDG), which is the most widely used PET radiopharmaceutical [J Nucl Med (1978), 19: 1154-1161]. However, a number of pitfalls and artefacts have been ascribed to FDG imaging and more continue to surface as the worldwide experience with FDG increases. The area most common for interpretative pitfalls with FDG is related to uptake in active skeletal muscle (Seminars in Nuclear Medicine, (2004), XXXIV, 2, pp.122-133). Many benign conditions can cause high accumulation of FDG creating the potential for false positive interpretation. Most of these artefacts are related to inflammatory, infective or granulomatous processes (Seminars in Nuclear Medicine, (2004), XXXIV, 2, pp.122-133, Seminars in Nuclear Medicine, (2004), XXXIV, 1, pp.56-69, (2004), J Nucl Med (2004), 45, pp. 695-700). Other tumours including mucosal associated lymphomas, small lymphocytic cell lymphoma, some neuroendocrine tumours, sclerotic bone metastases and renal cell carcinomas can be virtually inconspicuous due to low uptake or higher neighbouring background activity. Specifically related to PET-CT are pitfalls associated with breathing pattern differences between modalities, despite dedicated combined scanners (Seminars in Nuclear Medicine, (2004), XXXIV, 2, pp.122-133). For Prostate Cancer, uptake of [18F] FDG has been found to be low, likely due to the slow growing nature of many prostate tumours. As a consequence, in a large meta analysis (Gambhir SS, Czernin J, Schwimmer J, Silverman DH, Coleman RE, Phelps ME. A tabulated summary of the FDG PET literature. J Nucl Med. 2001 May; 42(5 Suppl):1S-93S.) only 57% of prostatic carcinomas could be detected by [18F] FDG.
Current methods for imaging prostate cancer are predominantly computed tomography (CT), magnetic resonance (MR) and ultrasound, however, these methods are only anatomic methods and do not have the same sensitivity than SPECT or PET. The radiolabelled monoclonal antibody [111In]-Prostascin™ is currently a marketed imaging agent for prostate cancer, but the images obtained from this agent are difficult to interpret (Lange et al., Urology, 2001, 57, 402-406, Haseman et al., Cancer Biother. Radiopharm., 2000, 15, 131-140).
Especially for the PET imaging of prostate cancer, but also for other type of cancers, tetra-substituted ammonium derivatives labelled with ¹¹C and ¹⁸F isotopes and based on choline structure have been described (e.g. JP09048747A, WO2001082864A (incl. US 2002061279A1)). Among these derivatives [methyl-(C-11)]choline (CH), [F-18]fluorocholine (FCH), [F-18]fluoroethylcholine (FEC), [F-18]fluoromethylethylcholine (FMEC) and [F-18]fluoropropylcholine (FPC) are the best investigated compounds (see Scheme A; e.g. Nuclear Medicine (2001), 42(12), 1805-1814). Also known are [18F]fluoro-dihydrotestosterone (FDHT), anti-1-amino-3-[18F]fluorocyclobutyl-1-carboxylic acid (FACBC), [11C]acetate, and 1-(2-deoxy-2-[18F]fluoro-L-arabinofuranosyl)-5-methyluracil (FMAU) (Scher et al., Eur. J. Nucl. Med. Mol. Imaging 2007, 34, 45-53; Rinnab et al., BJU Int., 2007, 100, 786-793; Reske at al., J. Nucl. Med., 2006, 47, 1249-1254; Zophel et al., Eur. J. Nucl. Med. Mol. Imaging 2004, 31, 756-759; Vees et al., BJU Int., 2007, 99, 1415-1420; Larson et al., J. Nucl. Med., 2004, 45, 366-373; Schuster et al., J. Nucl. Med., 2007, 48, 56-63; Tehrani et al., J. Nucl. Med., 2007, 48, 1436-1441). These different agents all work via different mechanisms, each with its own advantage or disadvantage.

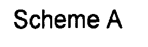

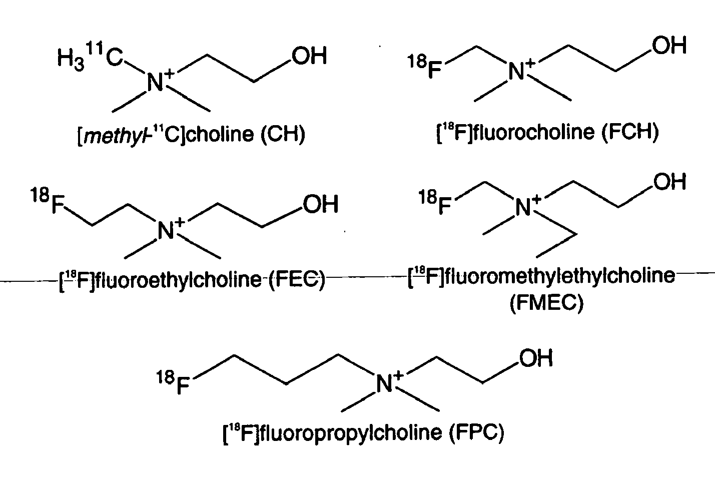

Initial clinical results of these above-mentioned compounds indicated somewhat better enrichment in prostate cancer tumours as compared to [18F]FDG. Nevertheless, imaging tracers with improved sensitivity/specificity profiles remain highly needed.

[11C]Methyl-L-methionine ([11C]Met) is able to illustrate tumor but is limited by the short half-life of the ¹¹C-isotope of 20.3 min (Mineura et al, Computerized Med Imaging and Graphics, vol 21, pp 63-66, 1997) and by the metabolism of Met in human body. Non-natural alicyclic amino acids such as 1-aminocyclobutane-1-[11C]carboxylic acid ([11C]ACBC) were found to be not metabolized (McConathy and Goodman, Cancer Metastasis Rev., vol 27, pp 555-573, 2008).

Radiolabeled amino acids have been explored for tumor imaging (Jager PL, Vaalburg W, Pruim J, de Vries EG, Langen KJ, Piers DA. Radiolabeled amino acids: basic aspects and clinical applications in oncology. J Nucl Med.2001 Mar;42(3):432-45.) to overcome the limitations seen for [18F]FDG. Initially, naturally occurring amino acids were labelled with ¹¹C such as [11C] valine, L-[11C]leucine, L-[11C]methionine, and structurally similar [18F]analogues. After uptake of these mainly neutral amino acids into the tumor cells predominantly via sodium independent L-type amino acid transporters, the retention of these tracers within the tumor cell is mainly due to protein synthesis. Limitations of radiolabeled naturally occurring amino acids include metabolic degradation via several pathways resulting in multiple radiolabeled metabolites which obscure the analysis of tumor uptake of the mother compound. Kinetic studies have suggested that amino acid transport rather than protein synthesis better reflect tumor proliferation. As a consequence, radiolabeled non-natural amino acids, such as 1-aminocyclobutane-1-[C11]carboxylic acid ([11C]ACBC), were explored, that can enter the cells via similar transport systems as natural amino acids, but are less prone to metabolic degradation (McConathy and Goodman, Cancer Metastasis Rev., vol 27, pp 555-573, 2008). Subsequently, [F18] derivatives and analogues of [11C]ACBC were described overcoming the logistical limitations of [11C]ACBC. Due to its short 20 minute half-life ¹¹C containing radiotracers require an on-site cyclotron, whereas ¹⁸F PET tracers, considering a half-life of 109 minutes, allow for off-site production and regional distribution.
Examples for [F18] labeled analogues of ACBC include 3-FACBC, 2-FACBC, and 2-FACPC (WO2009/129110A, WO2007/001958A, WO1997/017092A, WO2007/001940A). Among these, 3-FACBC and 2-FACPC have been studied in clinical trials with prostate cancer patients. While both compounds showed uptake into primary prostate cancer tumors (Schuster et al., abstract at SNM 2010), both differed remarkably in their pharmacokinetic properties. While 3-FACBC showed delayed renal elimination and high background in other organs such as liver and heart, 2-FACPC was excreted into the bladder more rapidly, nevertheless still showing high background signals for instance in the blood pool. Existing ¹⁸F analogues, such as 3-FACBC and 2-FACPC, allow for single step introduction of ¹⁸F starting from sulphamidate precursors described recently (Yu et al., Bioorg. Med. Chem. Lett., 2010, 20, 2140-2143). However, the synthesis remains challenging from a manufacturing and quality control perspective when taking into account the reported limited stability of the precursor for 3-FACBC (McConathy et al., Appl. Radiat. Isot., 2003, 58, 657; Shoup et al., J. Labeled Compd. Radiopharm., 1999, 42, 215) and the oily nature of the 2-FACBC and 2-FACPC precursors (WO2007/001940A, WO2009/129110A). The synthesis of the enantiopure 2-FACPC also requires a separation of the enantiomers by chiral HPLC on the tracer step (see W02009/129110A) which is unfavourable with regard to ease and reliability of manufacturing. Therefore it would be favourable to have enantiopure substance that is solid, and even more preferably, crystalline precursors that would allow for easier purification and thus higher purity of the compounds. In addition, crystalline substances e.g. precursors have a higher stability and longer shelf-life due to the more compact size and lower surface of the substances.

### Problem to be solved by the invention and its solution

Despite the aforementioned advances in finding suitable non-natural cyclic amino acids for tumor imaging and specifically for the imaging of prostate cancer, there remains a need for novel agents with improved signal. More specifically, the ¹⁸F labelled cyclic amino acids known so far, including the examples mentioned above, showed considerable uptake into non-tumor structures in particular into the liver.
In contrast, compounds of the present invention feature a rapid elimination of non tumor bound tracer while maintaining high tumor uptake, and in addition and unexpectedly showed reduced uptake into other organs.

### Summary

The invention relates to the subject matter referred to in the claims, i.e. fluoro labelled heterocyclic amino acid derivatives of the formula (I), (IIa) and (IIb), their precursors of the formula (IIIa) and (IIIb), their use and their preparation processes.

### Figures

Figure 1: ORTEP-plot (50% thermal ellipsoids) of example **6.**
Figure 2: Chromatogram of compound **48.**
Figure 3: Chromatogram of the final product **45** after acidic deprotection (C18 HPLC column).
Figure 4: Chromatogram of the final product **45** after acidic de protection (HILIC HPLC column with CAD Corona Detector).
Figure 5: Chromatogram of the final product **45** after acidic deprotection co-injected with the cold standard **15** (HILIC HPLC column with CAD Corona Detector).
Figure 6: Chromatogram of radiofluorinated intermediate **50** obtained from incorporation of [¹⁸F]fluoride into precursor **32** (Example 7).
Figure 7: Chromatogram of radiofluorinated intermediate **50** purified by SPE and obtained from precursor **32** (Example 7).
Figure 8: Chromatogram of one enantiomer of the final product **46** after acidic deprotection (C18 HPLC column).
Figure 9: Chromatogram of one enantiomer of the final product **46** after acidic deprotection (HILIC HPLC column with CAD Corona Detector).
Figure 10: Cell-uptake [14C] ACPC and compound **15** into DU145 prostate cancer cells.
Figure 11: PET/CT-imaging in DU145-tumor bearing mice with tracer **45.**
Figure 12: PET/CT-imaging in DU145-tumor bearing mice with tracer **46.**

### Description

In a **first** aspect, the invention is directed to compounds of the formula (I) 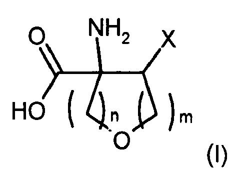 wherein X is Fluorine atom (F),
n = 1 or 2 and
m = 1 or 2 with the proviso that n and m are not both 2.

Formula I encompasses single isomers, diastereomers and enantiomers, mixtures thereof and pharmaceutically acceptable salts thereof.

Preferably, Fluorine atom (F) is an ¹⁸F or ¹⁹F isotope.

Preferably m = 1 and n = 1, m = 2 and n = 1 or m = 1 and n = 2.

More preferably m = 1 and n = 1 or 2. Even more preferably, m = 1 and n = 1.

In a **first** embodiment, the invention is directed to compounds of formula (I) wherein the Fluorine atom (F) is an ¹⁸F isotope.

In a **second** embodiment, the invention is directed to compounds of formula (I) wherein the Fluorine atom (F) is a ¹⁹F isotope.

Embodiments and preferred features can be combined together and are within the scope of the invention.

Invention compounds are but not limited to
(3*SR*,4*SR*)-3-Amino-4-[¹⁸F]fluorotetrahydrofuran-3-carboxylic acid 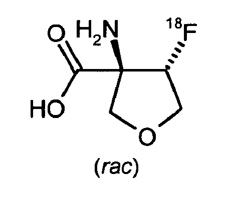 (3*SR*,4*SR*)-3-Amino-4-fluorotetrahydrofuran-3-carboxylic acid 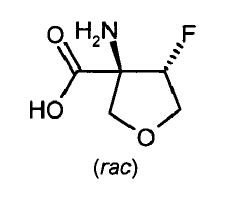 (3*R*,4*R*)-3-Amino-4-[¹⁸F]fluorotetrahydrofuran-3-carboxylic acid 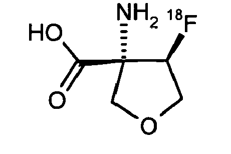 (3*R*,4*R*)-3-Amino-4-fluorotetrahydrofuran-3-carboxylic acid 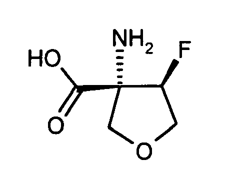 (3*RS*,4*RS*)-4-Amino-3-[¹⁸F]fluoro-3,4,5,6-tetrahydro-2H-pyran-4-carboxylic acid

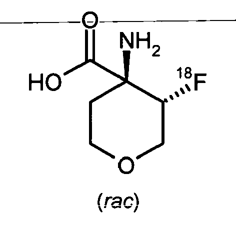

In a **second** aspect, the invention is directed to compounds of the formula (IIa) and (IIb) 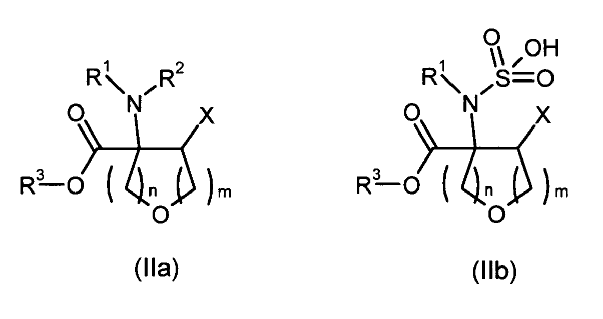 wherein X is Fluorine atom (F),
n = 1 or 2,
m = 1 or 2 with the proviso that n and m are not both 2,
R¹ = hydrogen or an N-protecting group,
R² = hydrogen or an N-protecting group and
R³ = hydrogen or an O-protecting group
or the group NR¹R² is a 1,3-dioxo-1,3-dihydro-2H-isoindol-2-yl (phthalimido) or an azido group;
with the proviso, that at least one of the substituents R¹, R² or R³ is not Hydrogen.

Formula II encompasses single isomers, diastereomers, enantiomers, mixtures thereof and pharmaceutically acceptable salts thereof.

Preferably, Fluorine atom (F) is an ¹⁸F or ¹⁹F isotope.

Preferably m = 1 and n = 1, m = 2 and n = 1 or m = 1 and n = 2.

More preferably m = 1 and n = 1 or 2. Even more preferably, m = 1 and n = 1.

The compounds of formula IIa and IIb are Fluoro-labeled compounds wherein the functional group(s) such as OH and NH₂ are protected or not protected with suitable protecting group(s) defined as R¹ to R³ respectively.

N-protecting groups are suitable for present invention and known in the art by the skilled person.
Preferably, N-protecting group is selected from the group comprising
Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn), p-Methoxybenzyl (PMB), 3,4-Dimethoxybenzyl (DMPM), Triphenylmethyl, p-Methoxyphenyl (PMP) and the moiety NR¹R² that is a 1,3-dioxo-1,3-dihydro-2H-isoindol-2-yl (phthalimido) or an azido group.
More preferably, N-protecting group is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn) and the moiety NR¹R² that is a 1,3-dioxo-1,3-dihydro-2H-isoindol-2-yl (phthalimido) or an azido group.
Even more preferably, N-protecting group is selected from the group comprising tert-Butoxycarbonyl (BOC) and the moiety NR¹R² that is a 1,3-dioxo-1,3-dihydro-2H-isoindol-2-yl (phthalimido).

Preferably, R² is N-protecting group and R¹ is Hydrogen or R¹ and R² are both N-protecting group.

O-protecting groups are suitable for present invention and known in the art by the skilled person.
O-protecting group is selected from the group of optionally substituted C₁-C₁₀-alkyl, optionally substituted allyl, and optionally substituted C₇-C₁₀-arylalkyl.

Preferably, C₁-C₁₀-alkyl is C₁-C₆-alkyl or C₇-C₁₀-alkyl. More preferably, C₁-C₆-alkyl is Methyl, Ethyl, Propyl, i-Propyl, Butyl or t-Butyl. Even more preferably, C₁-C₆-alkyl is Methyl or t-Butyl.

Preferably, C₇-C₁₀-arylalkyl is benzyl. Preferably, substituted C₇-C₁₀-arylalkyl is 4-Methoxybenzyl.

Preferably, O-protecting group is selected from the group comprising
Methyl, Ethyl, t-Butyl, Allyl, Benzyl, and 4-Methoxybenzyl. More preferably, O-protecting group is selected from the group comprising Methyl, Ethyl, t-Butyl, and Benzyl.

Preferably, R³ is O-protecting group.

In a **first** embodiment, the invention is directed to compounds of formula (IIa) wherein the Fluorine atom (F) is ¹⁸F isotope.

In a **second** embodiment, the invention is directed to compounds of formula (IIa) wherein the Fluorine atom (F) is ¹⁹F isotope.

In a **third** embodiment, the invention is directed to compounds of formula (IIb) wherein the Fluorine atom (F) is ¹⁸F isotope.

In a **fourth** embodiment, the invention is directed to compounds of formula (IIb) wherein the Fluorine atom (F) is ¹⁹F isotope.

Embodiments and preferred features can be combined together and are within the scope of the invention.

Invention compounds are but not limited to
N-(*tert-*Butoxycarbonyl)-*N*-[(3*SR*,4*SR*)-3-(*tert-*butoxycarbonyl)-4-fluorotetrahydrofuran -3-yl]sulfamic acid 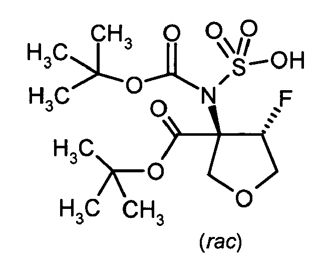 *N*-(*tert-*Butoxycarbonyl)-*N*-[(3*SR*,4*SR*)-3-(*tert-*butoxycarbonyl)-4-[¹⁸F]fluorotetrahydrofuran -3-yl]sulfamic acid 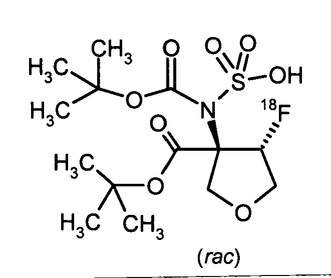 *N*-(*tert-*Butoxycarbonyl)-*N*-[(3*R*,4*R*)-3-(*tert-*butoxycarbonyl)-4-[¹⁸F]fluorotetrahydrofuran -3-yl]sulfamic acid 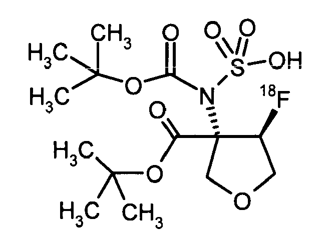 *N*-(*tert-*Butoxycarbonyl)-*N*-[(3*SR*,4*SR*)-4-(*tert-*butoxycarbonyl)-3-[¹⁸F]fluoro-3,4,5,6-tetrahydro -2*H*-pyran-4-yl]sulfamic acid

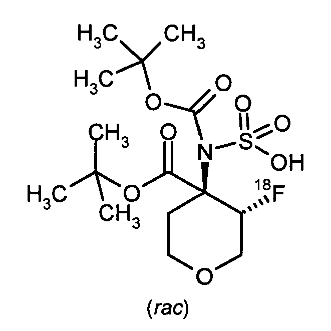

In a **third** aspect, the invention is directed to compounds of the formula (IIIa) and (IIIb) 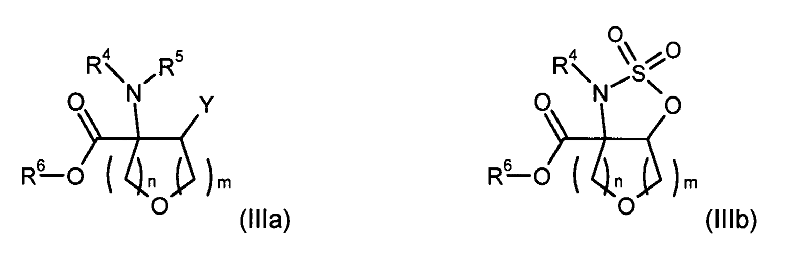 wherein Y is Leaving Group (LG),
n = 1 or 2,
m = 1 or 2 with the proviso that n and m are not both 2,
R⁴ = hydrogen or an N-protecting group,
R⁵ = N-protecting group,
R⁶ = O-protecting group
or the group NR⁴R⁵ is a 1,3-dihydro-2H-isoindol-2-yl (phthalimido) or an azido group.

Formulas (IIIa) and (IIIb) encompass single isomers, diastereomers, enantiomers, mixtures thereof pharmaceutically acceptable salts thereof.
The compounds of formulas (IIIa) and (IIIb) are compounds suitable for fluorolabeling wherein the functional group(s) such as OH and NH₂ are protected with suitable protecting group(s) such as R⁴, R⁵, and R⁶ respectively.

Preferably m = 1 and n = 1, m = 2 and n = 1 or m = 1 and n = 2.

More preferably m = 1 and n = 1 or 2. Even more preferably, m = 1 and n = 1.

O-protecting groups are suitable for present invention and known in the art by the skilled person.
Preferably, O-protecting group is selected from the group of optionally substituted C₁-C₁₀-alkyl, optionally substituted allyl, and optionally substituted C₇-C₁₀-arylalkyl.

Preferably, C₁-C₁₀-alkyl is C₁-C₆-alkyl or C₇-C₁₀-alkyl. More preferably, C₁-C₆-alkyl is Methyl, Ethyl, Propyl, i-Propyl, Butyl or t-Butyl. Even more preferably, C₁-C₆-alkyl is Methyl or t-Butyl.

Preferably, C₇-C₁₀-arylalkyl is benzyl. Preferably, substituted C₇-C₁₀-arylalkyl is 4-Methoxybenzyl.

Preferably O-protecting group is selected from the group comprising
Methyl, Ethyl, t-Butyl, Allyl, Benzyl, and 4-Methoxybenzyl. More preferably, O-protecting groups are selected from the group comprising Methyl, Ethyl, t-Butyl, and Benzyl.

R⁶ is O-protecting group.

In a **first** embodiment, the invention is directed to compounds of formula (IIIa) 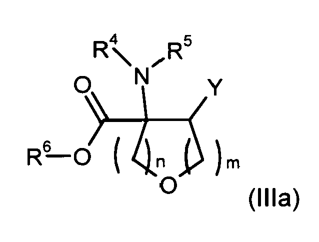 wherein Y is Leaving Group (LG),
n = 1 or 2,
m = 1 or 2 with the proviso that n and m are not both 2,
R⁴ = hydrogen or an N-protecting group,
R⁵ = N-protecting group and
R⁶ = O-protecting group
or the group NR⁴R⁵ is a 1,3-dioxo-1,3-dihydro-2H-isoindol-2-yl (phthalimido) or an azido group.

Formulas (IIIa) encompass single isomers, diastereomers, enantiomers, mixtures thereof and pharmaceutically acceptable salts thereof.

Preferably, the Leaving Group (LG) is sulphonate or halide. More preferably, the Leaving Group (LG) is sulphonate.

Preferably, halide is Chloro, Bromo or lodo.

Preferably, sulphonate is
Methylsulfonyloxy,
Trifluoromethylsulfonyloxy,
(4-Nitrophenyl)sulphonyloxy
Nonafluorbutylsulfonyloxy
(4-Bromophenyl)sulfonyloxy, or
(4-Methylphenyl)sulfonyloxy.
More preferably, the sulphonate is
Methylsulfonyloxy,
Trifluoromethylsulfonyloxy or
(4-Methylphenyl)sulfonyloxy.

N-protecting groups are suitable for present invention and known in the art by the skilled person.
Preferably N-protecting group is selected from the group comprising
Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn), p-Methoxybenzyl (PMB), 3,4-Dimethoxybenzyl (DMPM), Triphenylmethyl, p-Methoxyphenyl (PMP) and the moiety NR⁴R⁵ that is a 1,3-dioxo-1,3-dihydro-2H-isoindol-2-yl (phthalimido) or an azido group.

More preferably N-protecting group is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn) and the moiety NR⁴R⁵ that is a 1,3-dioxo-1,3-dihydro-2H-isoindol-2-yl (phthalimido) or an azido group.
Even more preferably, N-protecting group is selected from the group comprising tert-Butoxycarbonyl (BOC) and the moiety NR⁴R⁵ that is a 1,3-dioxo-1,3-dihydro-2H-isoindol-2-yl (phthalimido).

Preferably, R⁵ is N-protecting group and R⁴ is Hydrogen or R⁴ and R⁵ are both N-protecting group.

In a **second** embodiment, the invention is directed to compounds of formula (IIIb) 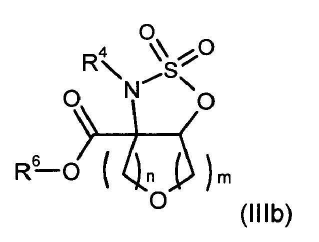 wherein
n = 1 or 2,
m = 1 or 2 with the proviso that n and m are not both 2,
R⁴ = N-protecting group and
R⁶ = O-protecting group.

Formulas (IIIb) encompass single isomers, diastereomers, enantiomers, mixtures thereof and pharmaceutically acceptable salts thereof.

N-protecting groups are suitable for present invention and known in the art by the skilled person.
Preferably, N-protecting group is selected from the group comprising
Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn), p-Methoxybenzyl (PMB), 3,4-Dimethoxybenzyl (DMPM), Triphenylmethyl, and p-Methoxyphenyl (PMP). More preferably, N-protecting group is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), and Benzyl (Bn).
Even more preferably, N-protecting group is tert-Butoxycarbonyl (BOC).

Embodiments and preferred features can be combined together and are within the scope of the invention.

Invention compounds are but not limited to
*tert-*Butyl (3*SR*,4*SR*)-3-[(*tert-*butoxycarbonyl)amino]-4-{[(trifluoromethyl)sulfonyl]oxy}-tetrahydrofuran-3-carboxylate 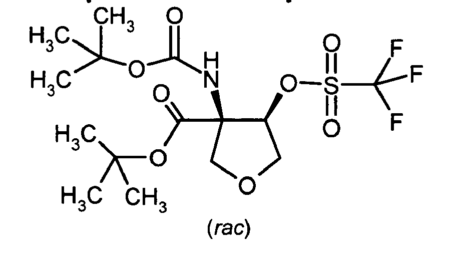 *tert*-Butyl (3*RS*,4*RS*)-3-[(*tert-*butoxycarbonyl)amino]-4-(mesyloxy)tetrahydrofuran-3-carboxylate 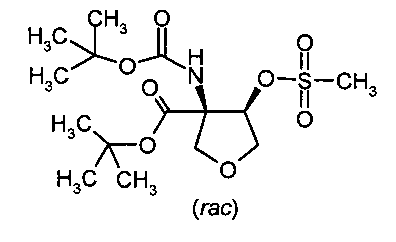 *tert-*Butyl (3*RS*,4*RS*)-3-[(*tert-*butoxycarbonyl)amino]-4-(tosyloxy)tetrahydrofuran-3-carboxylate 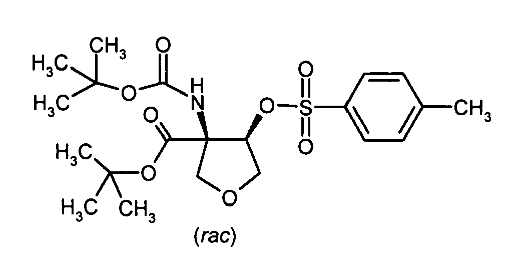 Methyl (3*RS*,4*RS*)-3-[(*tert-*butoxycarbonyl)amino]-4-(mesyloxy)tetrahydrofuran-3-carboxylate 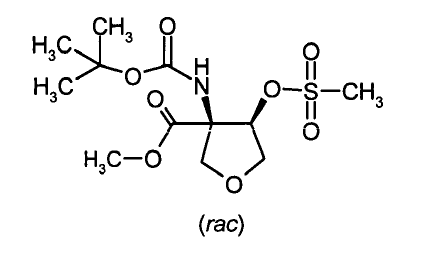 Methyl (3*RS*,4*RS*)-3-[(*tert-*butoxycarbonyl)amino]-4-(tosyloxy)tetrahydrofuran-3-carboxylate 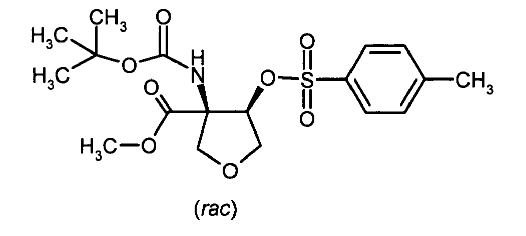 Di-tert-butyl (3a*RS*,6a*RS*)-3a,4,6,6a-tetrahydro-3*H*-furo[3,4*-d*]-1,2,3-oxathiazole-3,3a-dicarboxylate 2,2-dioxide 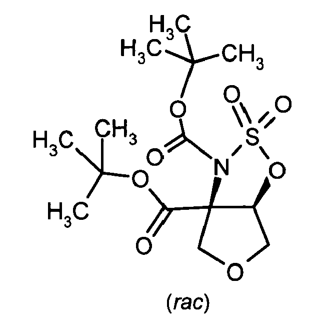 Di-tert-butyl (3a*S*,6a*S*)-3a,4,6,6a-tetrahydro-3*H*-furo[3,4*-d*]-1,2,3-oxathiazole-3,3a-dicarboxylate 2,2-dioxide 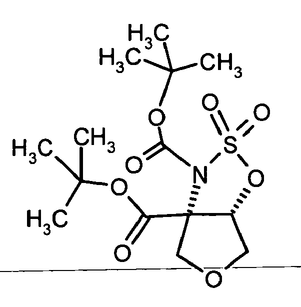 Di-*tert-*butyl (3a*RS*,7a*RS*)-3,3a,4,5,7,7a-hexahydropyrano[4,3*-d*]-1,2,3-oxathiazole-3,3a-dicarboxylate 2,2-dioxide 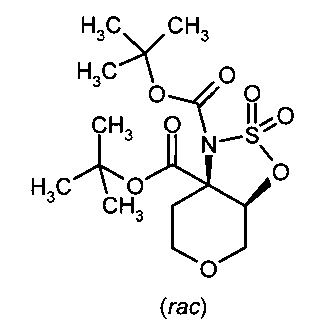 *tert-*Butyl (3*RS*,4*RS*)-4-[(*tert-*butoxycarbonyl)amino]-3-(mesyloxy)-3,4,5,6-tetrahydro-2H-pyran-4-carboxylate 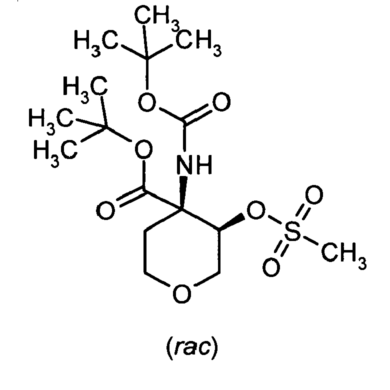 *tert-*Butyl (3*RS*,4*RS*)-4-[(*tert-*butoxycarbonyl)amino]-3-(tosyloxy)-3,4,5,6-tetrahydro-2H-pyran-4-carboxylate

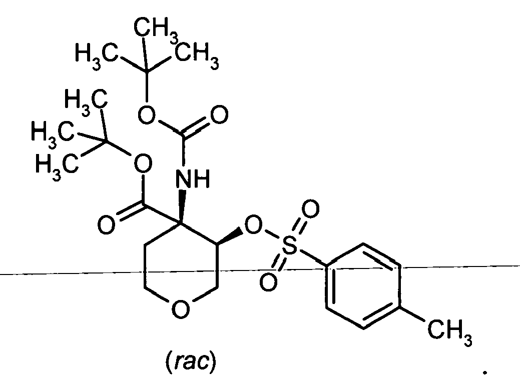

In a **fourth** aspect, the invention is directed to a composition comprising compounds of the formula (I), (IIa), (IIb), (IIIa), and/or (IIIb) or mixtures thereof and pharmaceutically acceptable carrier or diluent.
The person skilled in the art is familiar with auxiliaries, vehicles, excipients, diluents, carriers
or adjuvants which are suitable for the desired pharmaceutical formulations, preparations or compositions on account of his/her expert knowledge.
The administration of the compounds, pharmaceutical compositions or combinations
according to the invention is performed in any of the generally accepted modes of administration available in the art. Intravenous deliveries are preferred.

Generally, the pharmaceutical compositions according to the invention can be administered such that the dose of the active compound is in the range of 37 MBq (1 mCi) to 740 MBq (20 mCi). In particular, a dose in the range from 150 MBq to 370 MBq will be used.

In a **fifth** aspect, the invention is directed to method for obtaining compounds of formula (I), (IIa) and/or (IIb) or mixtures thereof.
The method of the invention is a fluoro-labeling method.
Preferably, the fluoro-labeling method concerns a method for labeling invention compounds with Fluorine atom (F) containing moiety wherein the Fluorine atom (F) containing moiety preferably comprises ¹⁸F or ¹⁹F.

More preferably, the Fluorine atom (F) containing moiety comprising ¹⁸F can be chelated complexes known to those skilled in the art, e.g. 4,7,13,16,21,24-Hexaoxa-1,10-diazabicyclo[8.8.8]-hexacosane K¹⁸F (crown ether salt Kryptofix K18F), 18-crown-6 ether salt K¹⁸F, K¹⁸F, H¹⁸F, KH¹⁸F₂, Rb¹⁸F, Cs¹⁸F, Na¹⁸F, or tetraalkylammonium salts of ¹⁸F known to those skilled in the art, e.g.[F-18] tetrabutylammonium fluoride, or tetraalkylphosphonium salts of ¹⁸F known to those skilled in the art, e.g.[F-18] tetrabutylphosphonium fluoride. Most preferably, the Fluorine atom (F) containing moiety is Cs¹⁸F, K¹⁸F, H¹⁸F, or KH¹⁸F₂.

More preferably, Fluorine atom (F) containing moiety comprises ¹⁹F. Even more preferably, the Fluorine atom (F) containing moiety is 4,7,13,16,21,24-Hexaoxa-1,10-diazabicyclo[8.8.8]-hexacosane KF (crownether salt Kryptofix KF), 1,4,7,10,13,16-hexaoxacyclooctadecane KF, KF, tetrabutylammonium fluoride, tetrabutylammonium dihydrogen trifluoride.

Preferably, the fluoro-labeling method is a fluoro-radiolabeling method.

Under the present invention, the method is a direct labelling method for obtaining compound of formula (I), (IIa) and/or (IIb) or mixture thereof.
The fluoro-labeling method comprises the steps
- Coupling compound of Formula (IIIa) or (IIIb) with Fluorine atom (F) containing moiety for obtaining compound of Formula (IIa) and/or (IIb),
- Optionally deprotecting compound of formula (IIa) and/or (IIb) for obtaining compound of Formula (I) and
- Optionally converting obtained compound into a pharmaceutically acceptable salts of inorganic or organic acids thereof, hydrates, complexes, esters, amides, and solvates thereof.

Preferably, the method is a direct labelling method for obtaining compound of formula (I), (IIa) and/or (IIb) or mixture thereof.
The fluoro-labeling method comprises the steps
- Coupling compound of Formula (IIIa) or (IIIb) with Fluorine atom (F) containing moiety wherein the Fluorine atom (F) containing moiety comprises ¹⁸F for obtaining compound of Formula (IIa) and/or (IIb),
- Optionally deprotecting group(s) of compound of formula (IIa) and/or (IIb) for obtaining compound of Formula (I) and
- Optionally converting obtained compound into a pharmaceutically acceptable salts of inorganic or organic acids thereof, hydrates, complexes, esters, amides, and solvates thereof.

Preferably, the method is a direct labelling method for obtaining compound of formula (I), (IIa) and/or (IIb) or mixture thereof.
The fluoro-labeling method comprises the steps
- Coupling compound of Formula (IIIa) or (IIIb) with Fluorine atom (F) containing moiety wherein the Fluorine atom (F) containing moiety comprises ¹⁹F for obtaining compound of Formula (IIa) and/or (IIb),
- Optionally deprotecting group(s) of compound of formula (IIa) and/or (IIb) for obtaining compound of Formula (I) and
- Optionally converting obtained compound into a pharmaceutically acceptable salts of inorganic or organic acids thereof, hydrates, complexes esters, amides, and solvates thereof.

The reagents, solvents and conditions which can be used for this fluorination are common and well-known to the skilled person in the field. See, *e.g.,* J. Fluorine Chem., 27 (1985):177-191. Preferably, the solvents used in the present method is DMF, DMSO, acetronitrile, DMA, or mixture thereof, preferably the solvent is DMSO.

A method for obtaining compound of formula (I), (IIa) and/or (IIb) or mixture thereof wherein compound of formula (I), (IIa), (IIb), (IIIa) and (IIIb) are as disclosed above wherein above embodiment and preferred features are herein enclosed.

In a **sixth** aspect, the invention is directed to compounds of formula (I), (IIa) or (IIb) for imaging in mammal.
The compounds of formula (I) is suitable for the manufacture of an imaging tracer for imaging proliferative diseases in mammal.
The compounds of formula (IIa) or (IIb) or mixture thereof is suitable for the manufacture of an imaging tracer for imaging proliferative diseases in mammal.

In other word, the invention is directed to the use of invention compounds of formula (I), (IIa) and/or (IIb) for the manufacture of an imaging tracer for imaging proliferative diseases.
The compounds of formula (I), (IIa) and (IIb) are herein defined as above and encompass all embodiments and preferred features herein enclosed.
Preferably, the invention compounds are compounds of formula (I), (IIa) or (IIb) wherein the Fluorine atom (F) is ¹⁸F isotope.
The imaging tracer is Positron Emission Tomography PET suitable imaging tracer or MicroPET.
The imaging comprises the step of PET imaging and is optionally preceded or followed by a Computed Tomography (CT) imaging or Magnetic Resonance Tomography (MRT) imaging.

The invention is also directed to a method for imaging or diagnosis proliferative diseases comprising the steps:
- Administrating to a mammal an effective amount of a compound comprising compounds of formula (I), (IIa) or (IIb),
- Obtaining an image of the mammal and
- Assessing the image.

Proliferative diseases are cancer characterised by the presence of tumor and/or metastases. Preferably, tumour are selected from the group of malignomas of the gastrointestinal or colorectal tract, liver carcinoma, pancreas carcinoma, kidney carcinoma, bladder carcinoma, thyroid carcinoma, prostrate carcinoma, endometrial carcinoma, ovary carcinoma, testes carcinoma, melanoma, small-cell and non-small-cell bronchial carcinoma, dysplastic oral mucosa carcinoma, invasive oral cancer; breast cancer, including hormone-dependent and hormone-independent breast cancer, squamous cell carcinoma, neurological cancer disorders including neuroblastoma, glioma, astrocytoma, osteosarcoma, meningioma, soft tissue sarcoma; haemangioma and endocrine tumours, including pituitary adenoma, chromocytoma, paraganglioma, haematological tumour disorders including lymphoma and leukaemias. Preferably, the tumor is a prostate carcinoma/prostate tumor.
Preferably, metastases are metastases of one of the tumours mentioned above. More preferably, metastases are metastases of a prostate carcinoma/prostate tumor.

Preferably, the invention compounds and use is for manufacturing a PET imaging tracer for imaging tumor in a mammal wherein the tumor is preferably a prostate carcinoma/prostate tumor.

In a **seventh aspect,** the invention is directed to the use of compounds of formula (I), (IIa), (IIb), (IIIa) or (IIIb) or mixture thereof for conducting biological assays and/or chromatographic identification. More preferably, the use relates to compounds of formula (I), (IIa) or (IIb) wherein the fluorine isotope is ¹⁸F or ¹⁹F, more preferably ¹⁹F.
Compounds of formula (I), (IIa) and (IIb), wherein the fluorine isotope is ¹⁹F, and (IIIa) or (IIIb) are useful as reference and/or measurement agent.
The compounds of formula (I), (IIa), (IIb), (IIIa) and (IIIb) are herein defined as above and encompass all embodiments and preferred features herein enclosed.

In a **eighth aspect,** the present invention provides a kit comprising a sealed vial containing a predetermined quantity of a compound having a Formula (I), (IIa), (IIb), (IIIa) or (IIIb) and pharmaceutically acceptable salts of inorganic or organic acids thereof, hydrates, complexes, esters, amides, and solvates thereof. Optionally the kit comprises a pharmaceutically acceptable carrier, diluent, excipient or adjuvant.

In a **ninth aspect,** the invention is directed to a method for monitoring tumor and/or metastases size by PET imaging a patient using compound of Formula (I), (IIa) and (IIb) or mixture thereof.
It has been surprisingly found that F18 compounds of present invention are potential PET radio tracer for imaging tumor and/or metastases in mammal. Rapid accumulation and retention of invention compounds into tumor and/or metastases allows for effective delineation of tumor and/or metastase and measurement of the tumor and/or metastases size from the obtained PET images. For the monitoring of tumor and/or metastases size, patients are PET imaged with invention compounds at least two times within a suitable time interval.
Monitoring of tumor and/or metastases size is obtained by imaging patient with invention compounds and comparing the tumor and/or metastases size obtained .

### Definitions

The terms used in the present invention are defined below but are not limiting the invention scope.
If chiral centers or other forms of isomeric centers are present in a compound according to the present invention, all forms of such stereoisomers, including enantiomers and diastereoisomers, are intended to be covered herein. Compounds containing chiral centers may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone. In cases wherein compounds may exist in tautomeric forms, such as keto-enol tautomers, each tautomeric form is contemplated as being included within this invention whether existing in equilibrium or predominantly in one form.

In the context of the present invention, preferred salts are pharmaceutically acceptable salts of the compounds according to the invention. The invention also comprises salts which for their part are not suitable for pharmaceutical applications, but which can be used, for example, for isolating or purifying the compounds according to the invention. Pharmaceutically acceptable salts of the compounds according to the invention include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalene disulphonic acid, formic acid, acetic acid, trifluoroacetic acid, propionic acid, lactic acid, tartaric acid, malic acid, citric acid, fumaric acid, maleic acid and benzoic acid.
Pharmaceutically acceptable salts of the compounds according to the invention also include salts of customary bases, such as, by way of example and by way of preference, alkali metal salts (for example sodium salts and potassium salts), alkaline earth metal salts (for example calcium salts and magnesium salts) and ammonium salts; derived from ammonia or organic amines having 1 to 16 carbon atoms, such as, by way of example and by way of preference, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

The term "N-protecting group" (amine-protecting group) as employed herein by itself or as part of another group is known or obvious to someone skilled in the art, which is chosen from but not limited to a class of protecting groups namely carbamates, amides, imides, N-alkyl amines, N-aryl amines, imines, enamines, boranes, N-P protecting groups, N-sulfenyl, N-sulfonyl and N-silyl, and which is chosen from but not limited to those described in the textbook Greene and Wuts, Protecting groups in Organic Synthesis, third edition, page 494-653, which is hereby incorporated herein by reference.

The term "O-protecting group" as employed herein refers to a carboxylic acid protecting group employed to block or protect the carboxylic acid functionality while the reactions involving other functional sites of the compound are carried out. Carboxy protecting groups are disclosed in Greene, "Protective Groups in Organic Synthesis" pp. 152-186 (1981), which is hereby incorporated herein by reference. Such carboxy protecting groups are well known to those skilled in the art, having been extensively used in the protection of carboxyl groups. Representative carboxy protecting groups are alkyl (e.g., methyl, ethyl or tertiary butyl and the like); arylalkyl, for example, phenethyl or benzyl and substituted derivatives thereof such as alkoxybenzyl or nitrobenzyl groups and the like.

Preferred O-protected compounds of the invention are compounds wherein the protected carboxy group is a loweralkyl, cycloalkyl or arylalkyl ester, for example, methyl ester, ethyl ester, propyl ester, isopropyl ester, butyl ester, sec-butyl ester, isobutyl ester, amyl ester, isoamyl ester, octyl ester, cyclohexyl ester, phenylethyl ester and the like or an alkanoyloxyalkyl, cycloalkanoyloxyalkyl, aroyloxyalkyl or an arylalkylcarbonyloxyalkyl ester.

The term "alkyl" as employed herein by itself or as part of another group refers to a C₁-C₁₀ straight chain or branched chain alkyl group such as, for example methyl, ethyl, propyl, isopropyl, butyl, isobutyl, *tert*-butyl, pentyl, isopentyl, neopentyl, heptyl, hexyl, decyl.

Preferably, alkyl is C₁-C₆ straight chain or branched chain alkyl or C₇-C₁₀ straight chain or branched chain alkyl. Lower alkyl is a C₁-C₆ straight chain or branched chain alkyl.

The term "arylalkyl" as employed herein by itself or as part of another group refers to alkyl substituted with an aryl wherein arylalkyl is C₇-C₁₀ alkyl carbon moiety that is optionally substituted as listed below. Preferred arylalkyl is benzyl or phenethyl. Preferably, , substituted arylalkyl is para methoxybenzyl.

The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl.

Whenever the term "substituted" is used, it is meant to indicate that one or more hydrogens on the atom indicated in the expression using "substituted" is / are replaced by one ore multiple moieties from the group comprising halogen, nitro, C₁-C₆-alkylcarbonyl, cyano, trifluoromethyl, C₁-C₆-alkylsulfonyl, C₁-C₆-alkyl, C₁-C₆-alkoxy and C₁-C₆-alkylsulfanyl, provided that the regular valency of the respective atom is not exceeded, and that the substitution results in a chemically stable compound, i. e. a compound that is sufficiently robust to survive isolation to a useful degree of purity from a reaction mixture, and formulation into a pharmaceutical composition.

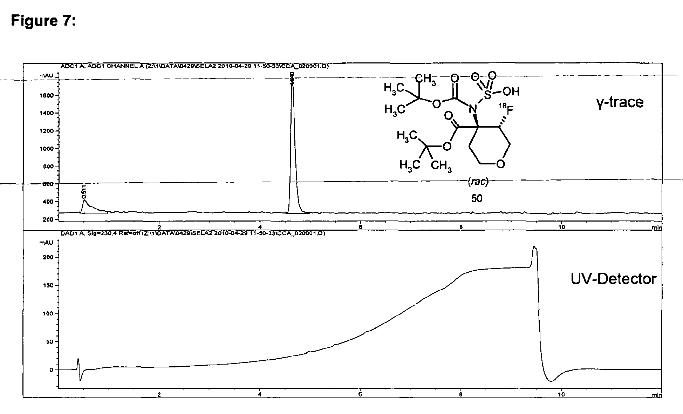

The term "leaving group" as employed herein by itself or as part of another group is known or obvious to someone skilled in the art, and means that an atom or group of atoms is detachable from a chemical substance by a nucleophilic agent. Examples are given e.g. in Synthesis (1982), p. 85-125, table 2 (p. 86; (the last entry of this table 2 needs to be corrected: "n-C₄F₉S(O)₂-O- nonaflat" instead of "n-C₄H₉S(O)₂-O- nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50, explicitly: scheme 4 pp. 25, scheme 5 pp 28, table 4 pp 30, Fig 7 pp 33).

The term "halide" as employed herein by itself or as part of another group is known or obvious to someone skilled in the art, and means fluoro, chloro, bromo, and iodo.

Unless otherwise specified, when referring to the compounds of formula the present invention per se as well as to any pharmaceutical composition thereof the present invention includes all of the hydrates, salts, and complexes.

### General synthesis of F-18 compounds

The radiofluorination reaction can be carried out, for example in a typical reaction vessel (e.g Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: dimethylsulfoxid and acetonitril as solvent and tetraalkyl ammonium and tetraalkyl phosphonium carbonate as base. Water and/or alcohol can be involved in such a reaction as co-solvent. The radiofluorination reactions are conducted for 1 to 60 minutes. Preferred reaction times are five to 50 minutes. Further preferred reaction times are 10 to 40 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (review: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

Precursors for ¹⁸F compounds of general formula I are e.g. tosylates, brosylates, nosylates, mesylates, triflates, nonaflates. (formula (IIIa)) which can be synthesized from the respective hydroxyl compounds according to methods known in the art (J. March, Advanced Organic Chemistry, 4th ed. 1992, John Wiley & Sons, pp 352ff), or sulphamidates (formula (IIIb)) which can be synthesized from the respective aminohydroxy compounds (see e.g. R. E. Meléndez et al., Tetrahedron 2003, 59, 2581-2616). More specifically, a hydroxy group being attached to a sp³ hybridized carbon atom can be converted to a leaving group by an activating agents like thionyl chloride (e.g. Organic and Biomolecular Chemistry; 4; 22; (2006); 4101 - 4112), phosphorus pentachloride (e.g. Bioorganic and Medicinal Chemistry; 16; 6; (2008); 3309-3320), methanesulfonyl chloride (e.g. Organic and Biomolecular Chemistry; English; 4; 24; (2006); 4514 - 4525), carbon tetrachloride / triphenylphosphine (Tetrahedron: Asymmetry; English; 19; 5; 2008; 577 - 583), hydrogen chloride (e.g. Russian Chemical Bulletin; English; 56; 6; 2007; 1119 - 1124), N-chloro-succinimide / S(CH₃)₂ (e.g. Bioscience, Biotechnology, and Biochemistry 72; 3; (2008); 851 - 855), hydrogen bromide (e.g. Journal of Labelled Compounds and Radiopharmaceuticals; 51; 1; (2008); 12 - 18), phosphorus tribromide (Journal of the American Chemical Society; 130; 12; (2008); 3726 - 3727), carbon tetrabromide / triphenylphosphine (e.g. Journal of the American Chemical Society; 130; 12; (2008); 4153 - 4157), N-bromo-succinimide / S(CH₃)₂ (e.g. Chemical Communications (Cambridge, United Kingdom); 1; (2008); 120 - 122), bromine / triphenylphosphine (e.g. Journal of the American Chemical Society; 130; 12; (2008); 4153 - 4157), N-bromo-succimide/S(CH₃)₂ (e.g. Chemical Communications (Cambridge, United Kingdom); 1; (2008); 120 - 122), Br₂/PPh₃(e.g. European Journal of Organic Chemistry; 9; (2007); 1510 - 1516), mesylchloride, tosylchloride, trifluormethylsulfonylchloride, nona-fluorobutylsulfonylchloride, (4-bromo-phenyl)sulfonylchloride, (4-nitro-phenyl)sulfonylchloride, (2-nitro-phenyl)sulfonylchloride, (4-isopropyl-phenyl)sulfonylchloride, (2,4,6-tri-isopropylphenyl)sulfonylchloride, (2,4,6-trimethyl-phenyl)sulfonylchloride, (4-tertbutyl-phenyl)sulfonylchloride, (4-methoxy-phenyl)sulfonylchloride, mesylanhydride, tosylanhydride, trifluormethylsulfonylanhydride, nona-fluorobutylsulfonylanhydride, (4-bromophenyl)sulfonylanhydride, (4-nitro-phenyl)sulfonylanhydride, (2-nitrophenyl)sulfonylanhydride, (4-isopropyl-phenyl)sulfonylanhydride, (2,4,6-tri-isopropylphenyl)sulfonylanhydride, (2,4,6-trimethyl-phenyl)sulfonylanhydride, (4-tertbutyl-phenyl)sulfonylanhydride, (4-methoxy-phenyl)sulfonylanhydride etc. In the case of sulphamidates of general formula (IIIb) the aminohydroxy compounds are reacted with sulphuryl chloride or thionyl chloride and then oxidized to give the corresponding sulphamidate (see e.g. R. E. Meléndez et al., Tetrahedron 2003, 59, 2581-2616). To those skilled in the art it is obvious that apart from the corresponding sulphuryl or thionyl chlorides also other electrophilic derivatives like imidazolides can be used.

Other precursors for ¹⁸F compounds of formula I are e.g. iodides and bromides and the like whose conversion to the respective fluorides is also known in the art (J. March, see above).

The synthesis of hydroxy compounds as starting materials for tosylates, brosylates, nosylates, mesylates, triflates, nonaflates, sulphamidates etc. comprises the deprotection of OH-protecting groups. As one of the very versatile protecting groups might be mentioned the benzyl protecting group. Many others are known in the art, see e.g. T.W. Greene and P.G.M. Wuts, Protective Groups in Organic Synthesis, 3rd ed, 1999, John Wiley & Sons, pp 17ff).

The amino acid moiety can be built up by a Strecker-type reaction from the corresponding carbonyl compound (for an overview about different amino acids synthesis strategies see e.g. J. Mulzer et al. in Organic Synthesis Highlights, VCH, Weinheim, 1991, p. 300 ff.). In particular, a ketone functionality can be converted to the corresponding hydantoine moiety by means of a Bucherer-Bergs-reaction (see e.g. R. Sarges et al., J. Med. Chem. 1990, 33, 1859-1865; K. Lavrador, Bioorg. Med. Chem. Lett. 1998, 8, 1629-1634) where upon the hydantoin is cleaved under basic conditions. To those skilled in the art it is also possible to cleave the hydantoin after a prior carbamate-protection of the amide- and/or imide-nitrogen atoms (see C. L. Wysong, J. Org. Chem. 1996, 61, 7650-7651).

The corresponding vicinal benzyloxy ketones are accessible by monoprotection of the corresponding diol followed by oxidation of the remaining free hydroxyl group to the corresponding ketone (R.C. Larock, Comprehensive Organic Transformations, VCH Publishers 1989, pp. 604-614). Moreover, the benzyloxy group can be introduced by α-oxidation of a ketone by means known to those skilled in the art (R. C. Larock, Comprehensive Organic Transformations, VCH Publishers 1989, pp. 488-489). On the other hand α-benzyloxy hydroxyl compounds can be accessed by reductive opening of a benzylidene acetal with a hydride donor agent like BH₃·S(CH₃)₂ (see e.g. J.-P. Surivet et al., Tetrahedron 1999, 55, 13011-13028).

The heterocycles used herein are, if not commercially available, accessible by cyclising condensation reactions or etherification reactions starting from the corresponding open-chained bis-sulphonates or halides with an oxygen nucleophile by means of a Finckelstein reaction (see e.g. R. C. Larock, Comprehensive Organic Transformations, VCH Publishers 1989, pp. 445-453).

The ¹⁹F-compounds can be synthesized according to the syntheses for the ¹⁸F-compounds (see above) or by deoxofluorination reactions (M. Hudlicky, Org. React. 1988, 35, 513; H. Vorbrüggen, Synthesis 2008, 8, 1165-1174.).

The radiofluorination reaction can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: acetonitrile, dimethylsulfoxide, sulfolane, dichloromethane, tetrahydrofuran, tertiary alcohols and o-dichlorobenzene as solvent and alkali metal with and without a suitable alkali metal chelating crown ether, tetraalkyl ammonium and tetraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (eview: Krasikowa, Synthesis Modules and Automation in F-18 labeling (2006), in: Schubiger P.A., Friebe M, Lehmann L., (eds), PET-Chemistry - The Driving Force in Molecular Imaging. Springer, Berlin Heidelberg, pp. 289-316) which allows an automated or semi-automated synthesis.

The term "electrophilisation-reagent" as employed herein by itself or as part of another group is known or obvious to someone skilled in the art, and is suited to convert a hydroxy group being attached to a sp³ hybridized carbon atom to a leaving group and which is chosen from but not limited to thionyl chloride (e.g. Organic and Biomolecular Chemistry; 4; 22; (2006); 4101 - 4112), phosphorus pentachloride (e.g. Bioorganic and Medicinal Chemistry; 16; 6; (2008); 3309-3320), methanesulfonyl chloride (e.g. Organic and Biomolecular Chemistry; English; 4; 24; (2006); 4514 - 4525), carbon tetrachloride / triphenylphosphine (Tetrahedron: Asymmetry; English; 19; 5; 2008; 577 - 583), hydrogen chloride (e.g. Russian Chemical Bulletin; English; 56; 6; 2007; 1119 - 1124), N-chloro-succinimide/ dimethylsulfide (e.g. Bioscience, Biotechnology, and Biochemistry 72; 3; (2008); 851 - 855), hydrogen bromide (e.g. Journal of Labelled Compounds and Radiopharmaceuticals; 51; 1; (2008); 12 - 18), phosphorus tribromide (Journal of the American Chemical Society; 130; 12; (2008); 3726 - 3727), carbon tetrabromide / triphenylphosphine (e.g. Journal of the American Chemical Society; 130; 12; (2008); 4153 - 4157), N-bromo-succimide/SMe₂ (e.g. Chemical Communications (Cambridge, United Kingdom); 1; (2008); 120 - 122), bromine / triphenylphosphine (e.g. Journal of the American Chemical Society; 130; 12; (2008); 4153 - 4157), N-bromo-succimide/SMe₂ (e.g. Chemical Communications (Cambridge, United Kingdom); 1; (2008); 120 - 122), Br₂/PPh₃ (e.g. European Journal of Organic Chemistry; 9; (2007); 1510 - 1516), mesylchloride, tosylchloride, trifluormethylsulfonylchloride, nona-fluorobutylsulfonylchloride, (4-bromo-phenyl)sulfonylchloride, (4-nitro-phenyl)sulfonylchloride, (2-nitro-phenyl)sulfonylchloride, (4-isopropyl-phenyl)sulfonylchloride, (2,4,6-tri-isopropylphenyl)sulfonylchloride, (2,4,6-trimethyl-phenyl)sulfonylchloride, (4-*tert*butyl-phenyl)sulfonylchloride, (4-methoxy-phenyl)sulfonylchloride, mesylanhydride, tosylanhydride, trifluormethylsulfonylanhydride, nona-fluorobutylsulfonylanhydride, (4-bromophenyl)sulfonylanhydride, (4-nitro-phenyl)sulfonylanhydride, (2-nitrophenyl)sulfonylanhydride, (4-isopropyl-phenyl)sulfonylanhydride, (2,4,6-tri-isopropylphenyl)sulfonylanhydride, (2,4,6-trimethyl-phenyl)sulfonylanhydride, (4-*tert*butyl-phenyl)sulfonylanhydride, (4-methoxy-phenyl)sulfonylanhydride, ect.

Scheme 1 shows the synthesis of the racemic spirohydantoin **4** starting from diol **1**. The enantiomeric hydantoins were separated by chiral HPLC.

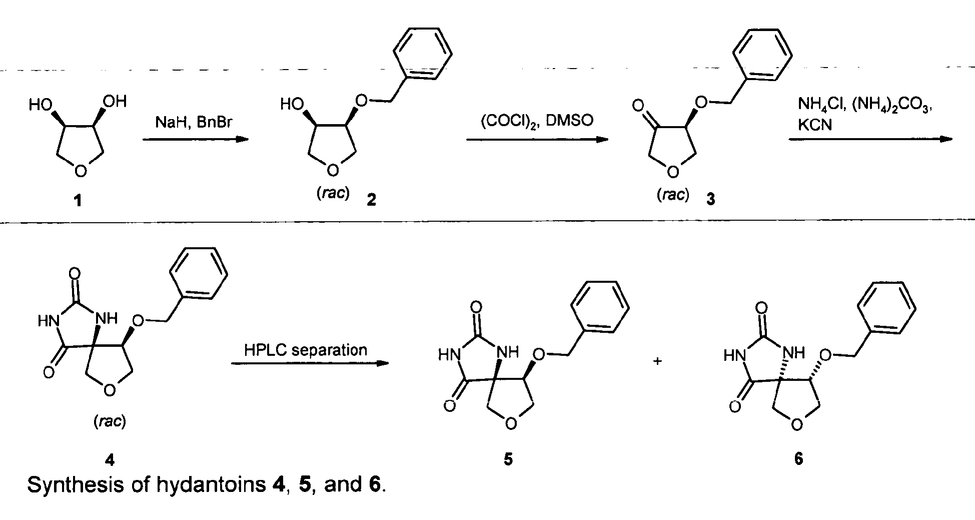

Scheme 2 shows the transformation of the racemic spirohydantoin **4** into ester **9** and **10** and further transformation of **10** into sulphamidate precursor **11** and the transformation of the latter into the protected fluoroaminoacid **12** and further to the free fluoroaminoacid **13.** The enantiopure compounds were synthesized accordingly starting from the enantiopure spirohydantoins **5** and **6.**

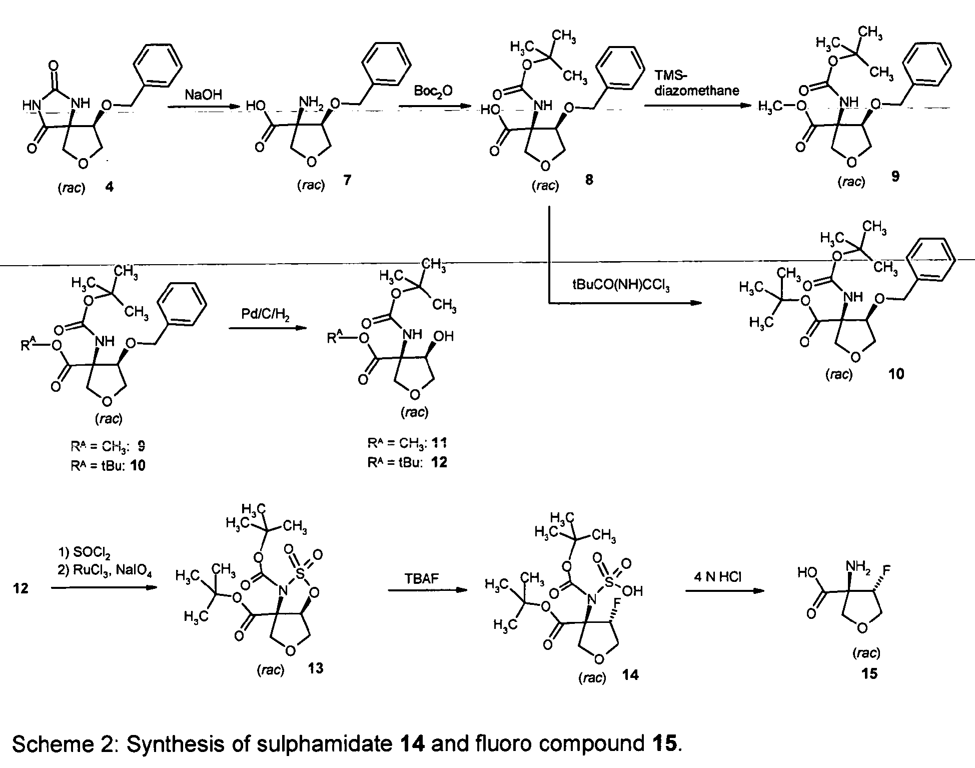

Scheme 3 shows the transformation of alcohols **9** and **10** to the corresponding sulphonates **16-20.**

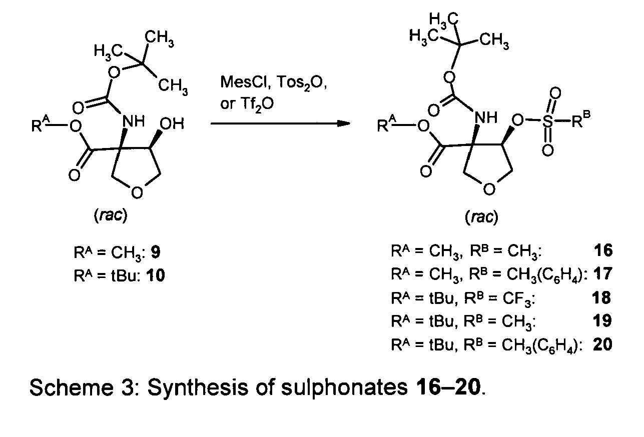

Scheme 4 shows the synthesis of the racemic diastereomeric benzyloxy compounds **28** and **29** starting from tetrahydropyran-4-on **(21).** The separated diasteromers are then transformed into the corresponding free alcohols **30** and **31. 30** is then further converted into the corresponding sulphamidate **32** and sulphonates **33** and **34.**

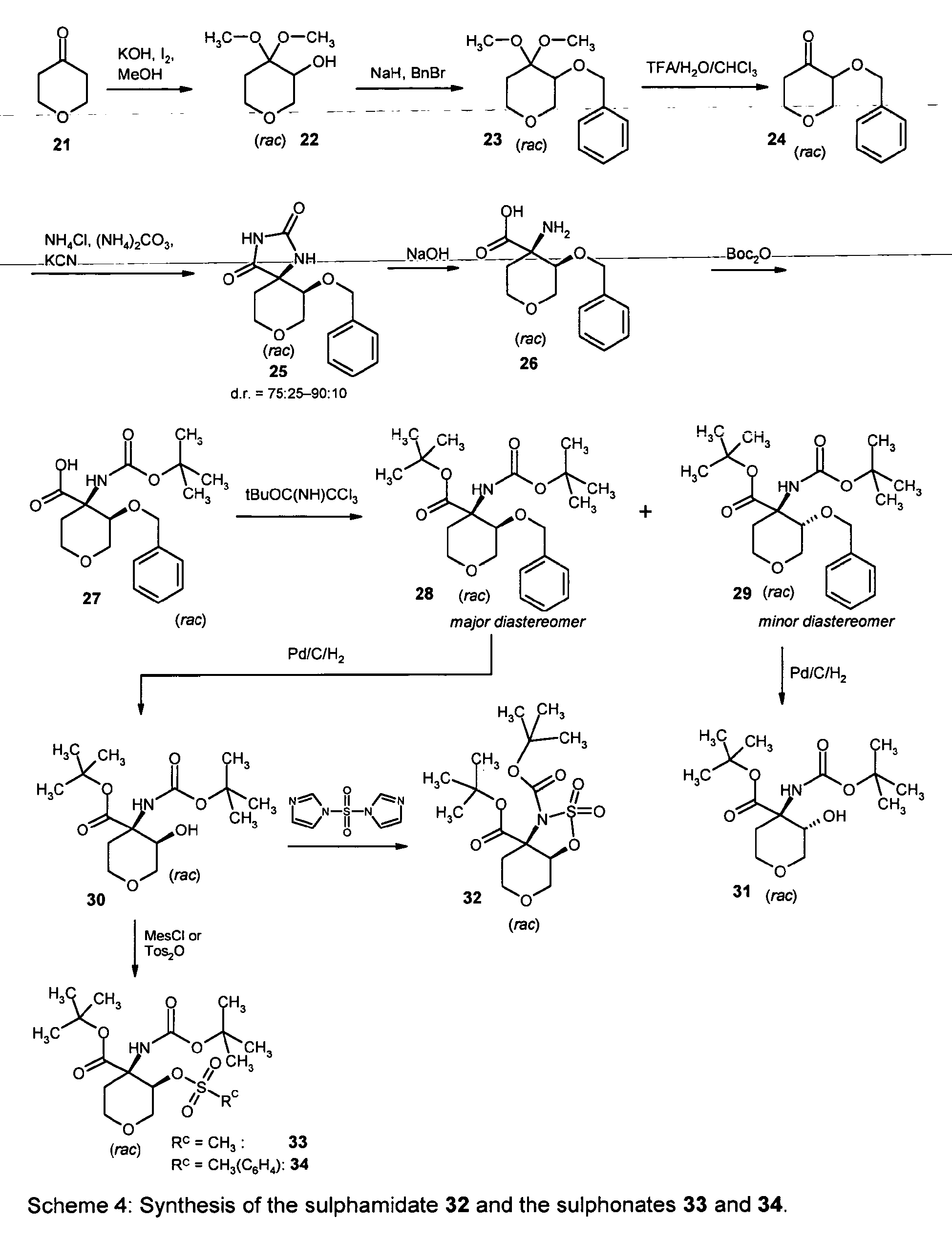

The regioisomeric fluoro-amino-tetrahydropyrane-amino-carboxylic acid **41** can be synthesized accordingly as shown in scheme 5 starting from tetrahydropyran-3-on **(35).**

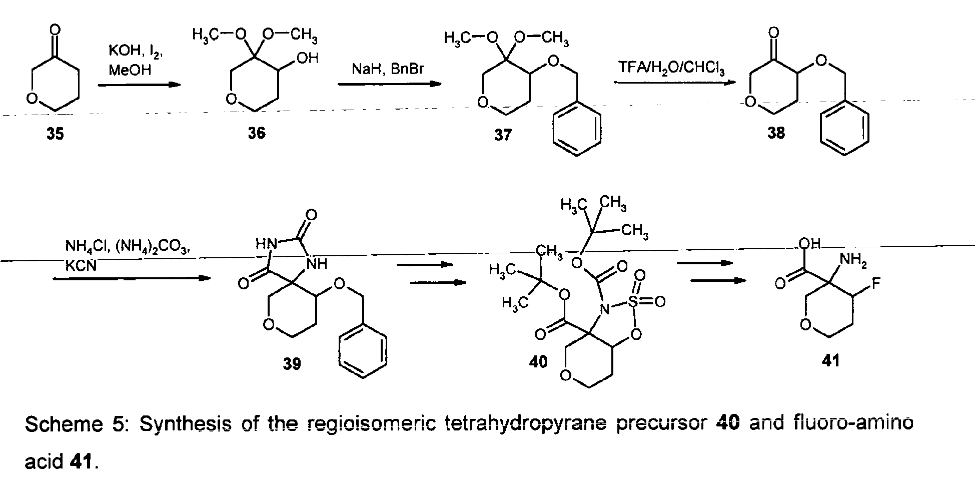

The 18F-compounds is synthesized by reaction of precursors of type (IIIa) or (IIIb) with [18F]fluoride followed by a deprotection step. The radiofluorination employing sulphonate precursors of type (IIIa) is shown in scheme 6.

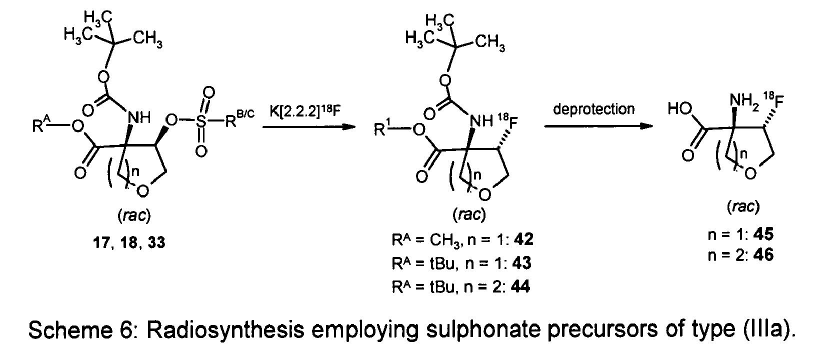

The radiofluorination employing sulphamidate precursors of type (IIIb) is shown in scheme 7.

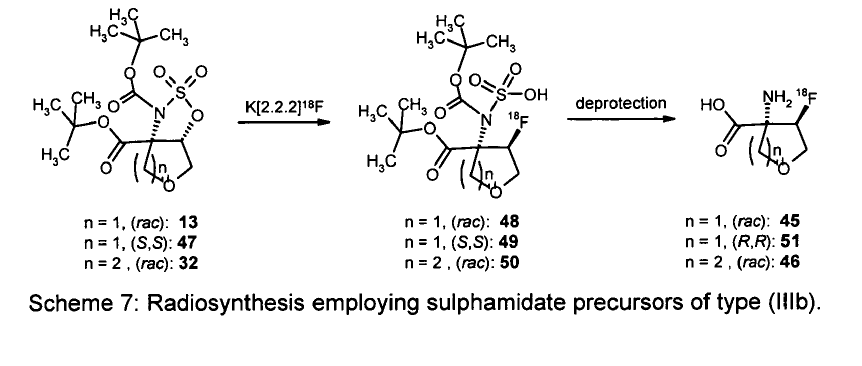

### Experimental Section

### Abbreviations

| | |
|---|---|
| ACN | Acetonitrile |
| br | broad signal (in NMR) |
| d | doublet |
| dd | doublet of doublet |
| DMA | *N*,*N*-dimethylacetamide |
| DMF | *N*,*N*-dimethylformamide |
| DMSO | dimethylsulphoxide |
| dt | doublet of triplet |
| ESI | Electrospray ionisation |
| h | hour |
| HPLC | High Pressure Liquid Chromatography |
| K₂₂₂ | Kryptofix 2.2.2 |
| m | multiplet |
| min | minute |
| MBq | MegaBequerel |
| MS | Mass spectrometry |
| MeOH | methanol |
| MTBE | methyl tert-butyl ether |
| NMR | Nuclear magnetic resonance spectroscopy : chemical shifts (δ) are given in ppm. |
| r.t. | room temperature |
| s | singlet |
| t | triplet |
| TBAOH | Tetrabutylammonium hydroxide |
| THF | tetrahydrofuran |
| TFA | trifluoro acetic acid |

**General:** All solvents and chemicals were obtained from commercial sources and used without further purification. Anhydrous solvents and inert atmosphere (nitrogen or argon) were used if not stated otherwise. The preceding table lists the abbreviations used in this paragraph and in the Intermediates and Examples sections as far as they are not explained within the text body. NMR peak forms are stated as they appear in the spectra, possible higher order effects have not been considered.
Reactions employing microwave irradiation can be run with a commercial laboratory microwave oven for organic syntheses, such as the Biotage Initiator® microwave, optionally equipped with a robotic unit. Reactions were monitored by methods known to the person skilled in the art, such as thin-layer chromatography on suitable stationary phases, such as silica gel coated plates of aluminium or glass, or HPLC/MS analyses.
The compounds and intermediates produced according to the methods of the invention may require purification. Purification of organic compounds is well known to the person skilled in the art and there may be several ways of purifying the same compound. In some cases, no purification may be necessary. In certain cases, the compounds may be purified by crystallization. In some cases, impurities may be removed by trituration using a suitable solvent. In some cases, the compounds may be purified by column chromatography, or preparative HPLC according to the preparative HPLC methods listed below.
Column chromatography, as used hereinafter, typically refers to preparative liquid chromatography on a suitable stationary phase, such as commercial silica gel or prepacked silica gel cartridges, *e.g.* from Separtis such as Isolute® Flash silica gel or Isolute® Flash NH₂ silica gel in combination with *e.g*. an automated column chromatography system, and eluents such as gradients of hexane/EtOAc or dichloromethane/ethanol. Said automated chromatography systems are known to the person skilled in the art and are commercially available (*e.g*. FlashMaster II® by Argonaut/Biotage, SP4® by Biotage, Isolera Four® by Biotage, ISCO Companion®, and the likes).

### Example 1

### 4) (5RS,9RS)-9-(Benzyloxy)-7-oxa-1,3-diazaspiro[4.4]nonane-2,4-dione

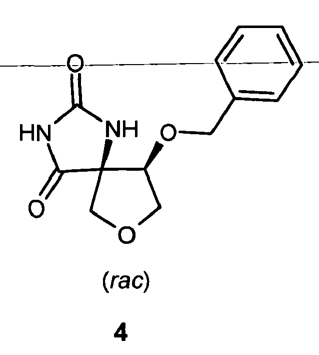

A supension of NaH (34.4 g, 861 mmol, 60% in mineral oil) in THF (700 mL) was cooled to 0°C and a solution of (*meso*)-tetrahydrofuran-3,4-diol (**1**) (89.6 g, 861 mmol) in DMSO (350 mL) was added carefully (-10 mL/min). After the evolution of hydrogen has ceased, the cooling bath was removed and the mixture was stirred mechanically for two hours at ambient temperature. The reaction vessel was cooled again with an ice bath and benzyl bromide (102 mL, 861 mmol) in THF (150 mL) was added (-17 mL/min). After stirring overnight the reaction was quenched in portions with water (overall 2.4 L) and extracted into diethylether (overall 2.4 L). The combined organic layers were dried over magnesium sulphate and the solvents were evaporated to give 163 g of crude alcohol **2**, which was used without further purification.

Oxalyl chloride (68.3 mL, 807 mmol) in dichloromethane (1000 mL) was cooled with dry ice in acetone. DMSO (115 mL, 1.61 mol) in dichloromethane (250 mL) was added over 75 min while the temperature of the reaction mixture was kept below -60°C. Prior to the addition of the crude alcohol **2** (163 g, ∼672 mmol) over 45 min the solution was stirred for 15 min at -70°C. After 45 min triethylamine (468 mL, 3.36 mol) was added. The resulting suspension was warmed slowly overnight to ambient temperature. Hydrochloric acid (1000 mL, 3 M) was added and the phases were separated. The aqueous layer was extracted with dichloromethane (2 x 300 mL) and the combined organic layers were dried over magnesium sulphate. The solvent was evaporated to give 163 g of crude ketone **3** which was used without further purification.

171 g of crude ketone **3** (-700 mmol) in methanol (2 L) were added at ambient temperature to a solution of ammonium carbonate (741 g, 7.71 mol) and ammonium chloride (165 g, 3.09-mol) in water (3L). After 30 min potassium cyanide (226 g, 3.47 mol) was added and the reaction mixture was heated to 50°C for 17 h. The chilled solution was extracted in portions with ethyl acetate (overall 6 I). The combined organic layers were evaporated and the hydantoin **4** was crystallized twice from isopropanol. The product was isolated in a diastereomeric purity of>93:7.
Yield: 44.5 g, 20 % over three steps from diol **1**.
¹H-NMR (400MHz, DMSO-d₆): δ [ppm]= 3.65 (dd, 1H), 3.77 (d, 1H), 3.95 (d, 1H), 4.02 (dd, 1H), 4.19 (t, 1H), 4.48 (s, 2H), 7.28 - 7.37 (m, 5H), 8.50 (s, 1H), 10.82 (br. s., 1H).
¹³C-NMR (101MHz, DMSO-d₆): δ [ppm]= 69.0 ppm, 70.8, 71.8, 73.6, 80.8, 127.6, 127.7, 128.3, 137.5, 156.6, 176.0.
MS (ESIpos): m/z = 263 [M+H]⁺

### 5) (5R,9R)-9-(Benzyloxy)-7-oxa-1,3-diazaspiro[4.4]nonane-2,4-dione

### 6) (5S,9S)-9-(Benzyloxy)-7-oxa-1,3-diazaspiro[4.4]nonane-2,4-dione

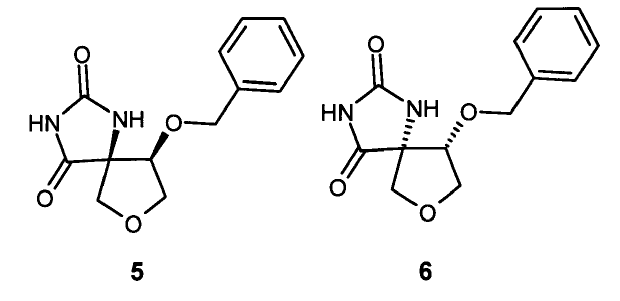

**5** and **6** were isolated from racemic **4** upon preparative HPLC separation. The absolute stereoconfiguration was determined by X-ray diffractometry from **6.**

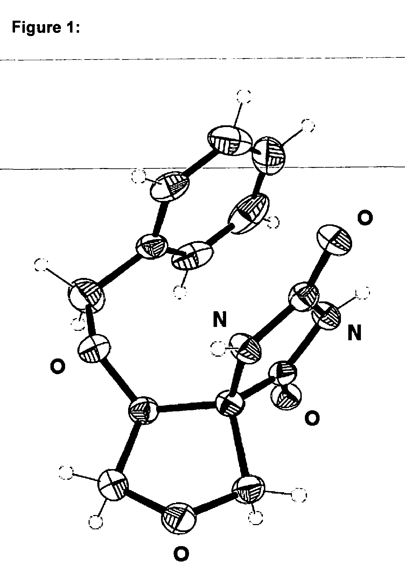

Figure 1 shows an ORTEP-plot from **6.**

### Preparative HPLC:

COLUMN: Chiralpak AD 20µm 210x51 mm
SOLVENT: Hexan / Ethanol 80:20
FLOW: 200 mL/min
TEMPERATURE: room temperature
DETECTION: UV 210 nm

### Analytical HPLC:

COLUMN: Chiralpak AD-H 5µm 150x4.6 mm
SOLVENT: Hexan / Ethanol 80:20
FLOW: 1.0 mL/min
TEMPERATURE: room temperature
DETECTION: UV 210 nm
**5:** *t*_{R} = 11.57 min, [a]_{D}²⁰ = +44.1° (589 nm), *c* 1.0, (MeOH)
**6:** *t*_{R} = 14.16 min, [a]_{D}²⁰ = -41.9° (589 nm), *c* 1.0, (MeOH).

### 10) tert-Butyl (3RS,4RS)-4-(benzyloxy)-3-[(tert-butoxycarbonyl)amino]tetrahydrofuran-3-carboxylate

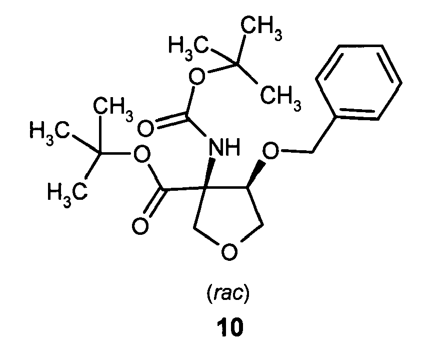

A solution of hydantoin **4** (8.72 g, 33.2 mmol) in 3 M aqueous sodium hydroxide solution (150 mL) was stirred under reflux for 3 days. After cooling to r.t. the pH of the mixture was adjusted to 6-7 with concentrated hydrochloric acid and then lyophilised.

The crude amino acid **7** was suspended in MeOH/triethylamine (9:1, 500 mL) at r.t. and di-tert-butyl-dicarbonate (10.9 g, 49.9 mmol) was added. After stirring for 24 h at r.t. the solids were filtered oof and the filtrate was concentrated under reduced pressure. The residue was taken up in ethyl acetate (100 mL) and 2 M (100 mL) aqueous sodium hydroxide solution and stirred vigorously at r.t. for 10 min. After that the pH was adjusted to 6-7 and the layers were separated. The aqueous phase was extracted twice with ethyl acetate, then acidified to pH 2 and again extracted with ethyl acetate. The combined organic phases were washed with 2 N hydrochloric acid, dried over sodium sulphate, filtered, and concentrated under reduced pressure.

The crude *N*-Boc-protected amino acid **8** was suspended in dichloromethane (200 mL) at r.t. and tert-butyl 2,2,2-trichloroacetimidate (13.0 mL, 69.7 mmol) was added. After 1 day more tert-butyl 2,2,2-trichloroacetimidate (2.6 mL, 13.9 mmol) was added and the mixture stirred again for 1 day. Then the mixture was filtered and concentrated under reduced pressure. The crude product was purified by column chromatography (SiO₂, hexane/ethyl acetate gradient).
Yield: 4.89 g, 37 % over three steps from hydantoin **4.**
¹H-NMR (400MHz, CHLOROFORM-d): δ [ppm]= 1.47 (s, 9H), 1.49 (s, 9H), 3.65 (dd, 1H), 4.04 (dd, 1H), 4.13 (d, 1H), 4.23 (t, 1H), 4.46 - 4.58 (m, 2H), 4.72 (d, 1H), 5.67 (s, 1H), 7.28 - 7.43 (m, 5H).
MS (ESIpos): m/z = 394 [M+H]⁺

### 9) Methyl (3RS,4RS)-4-(benzyloxy)-3-[(tert-butoxycarbonyl)amino]tetrahydrofuran-3-carboxylate

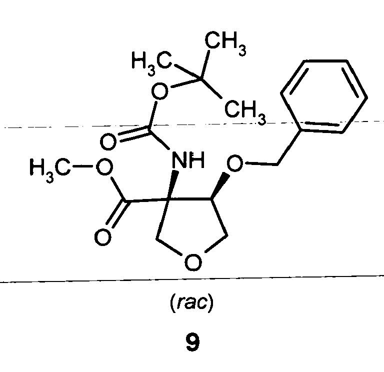

This compound was synthesized according to the procedure for tert-butyl ester **10**. However, for the last step of this synthesis the crude free acid **8** (1.50 g, 4.45 mmol) was added to a mixture of tetrafluoroboric acid diethylether complex (129 µL, 0.94 mmol) in toluene/methanol 9:1 (80 mL) at r.t.. After stirring for 2 min at this temperature trimethylsilyldiazomethane (8.89 mL, 17.8 mmol) was added and the mixture stirred for 3.5 h at r.t. and for 14 h at 4°C. Then the reaction mixture was concentrated under reduced pressure and the crude product was purified by column chromatography (SiO₂, hexane/ethyl acetate gradient).
Yield: 1.18 g, 23 % over three steps from hydantoin **4.**
¹H-NMR (300MHz, CHLOROFORM-d): δ [ppm]= 1.46 (s, 9H), 3.56 - 3.69 (m, 1H), 3.78 (s, 3H), 4.01 - 4.08 (m, 1H), 4.08 - 4.18 (m, 1H), 4.20 - 4.28 (m, 1H), 4.47 - 4.60 (m, 2H), 4.69 (d, 1H), 5.70 (br. s., 1H), 7.29 - 7.44 (m, 5H).
MS (ESIpos): m/z = 352 [M+H]⁺

### 12) tert-Butyl (3RS,4RS)-3-[(tert-butoxycarbonyl)amino]-4-hydroxytetrahydrofuran-3-carboxylate

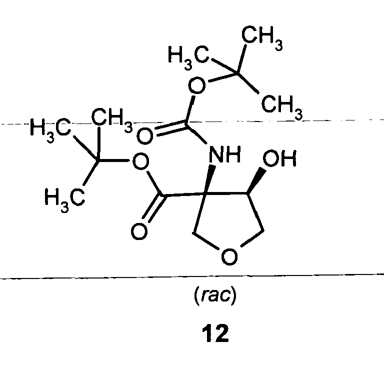

A mixture of **10** (4.89 g, 12.4 mmol) and palladium (500 mg, 10 % on charcoal) in methanol (300 mL) was stirred for 14 h at r.t. under a hydrogen atmosphere. After that another portion of palladium (500 mg, 10 % on charcoal) was added and the mixture stirred for further 3 h under a hydrogen atmosphere at r.t.. Then the mixture was filtered over Celite, the filter cake rinsed thoroughly with methanol and the filtrate was concentrated under reduced pressure.
Yield: 3.58 g, 95 %.
¹H-NMR (300MHz, CHLOROFORM-d): δ [ppm]= 1.46 (s, 9H), 1.48 (s, 9H), 2.78 (d, 1H), 3.74 (dd, 1H), 4.01 (d, 1H), 4.14 (dd, 1H), 4.32 (d, 1H), 4.54 (br. s., 1H), 5.54 (s, 1H).
MS (ESIpos): m/z = 304 [M+H]⁺

### 52) tert-Butyl (3SR,4SR)-3-[(tert-butoxycarbonyl)amino]-4-hydroxytetrahydrofuran -3-carboxylate

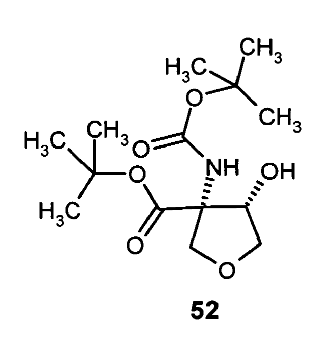

The compound was prepared according to the racemic compound **12** starting from **6.**
[a]_{D}²⁰= -13.7°± 0.11°, *c*1 (methanol).

### 11) Methyl (3RS,4RS)-3-[(tert-butoxycarbonyl)amino]-4-hydroxytetrahydrofuran-3-carboxylate

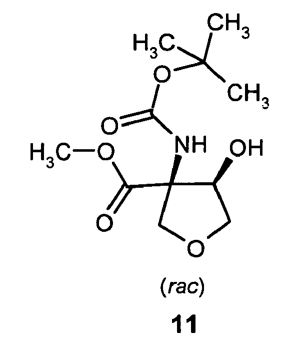

A mixture of **9** (1.18 g, 3.36 mmol) and palladium (150 mg, 10 % on charcoal) in methanol (200 mL) was stirred for 14 h at r.t. under a hydrogen atmosphere. After that another portion of palladium (150 mg, 10 % on charcoal) was added and the mixture stirred for one further day under a hydrogen atmosphere at r.t.. Then the mixture was filtered over Celite, the filter cake rinsed thoroughly with methanol and the filtrate was concentrated under reduced pressure. The crude product was purified by column chromatography (SiO₂, hexane/ethyl acetate gradient).
Yield: 740 mg, 80 %.
¹H-NMR (300MHz, CHLOROFORM-d): δ [ppm]= 1.40 (br. s., 9H), 2.80 (br. s., 1H), 3.72 (dd, 1H), 3.77 - 3.81 (m, 3H), 3.95 - 4.08 (m, 1H), 4.18 (dd, 1H), 4.41 (d, 1H), 4.57 (br. s., 1H), 5.62 (s, 1H).
MS (ESIpos): m/z = 262 [M+H]⁺

### 13) Di-tert-butyl (3aRS,6aRS)-3a,4,6,6a-tetrahydro-3H-furo[3,4-d]-1,2,3-oxathiazole -3,3a-dicarboxylate 2,2-dioxide

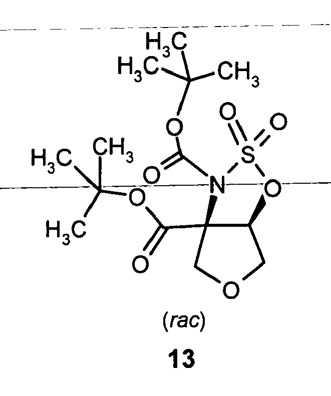

A mixture of **12** (50.0 mg, 0.16 mmol) and triethylamine (92 µL, 0.66 mmol) in dichloromethane (6 mL) was added dropwise to a solution of thionyl chloride (15.6 µL, 0.21 mmol) in dichloromethane (6 mL) at -78°C. After stirring for 3 h at this temperature more thionyl chloride (6 µL, 82 µmol) was added and the reaction mixture was warmed to r.t.. After 30 min the mixture was concentrated under reduced pressure at 30°C and the crude sulphamidite was used directly in the next step.

The crude sulphamidite was dissolved in acetonitrile (5 mL) at 0°C and RuCl_{3·}H₂O (1.9 mg, 8.2 µmol), sodium periodate (146 mg, 0.68 mmol) and water (0.8 mL) were added. The mixture stirred for 1.5 h at 0°C and for 0.5 h at r.t.. After that the mixture was diluted with ethyl acetate (20 mL) and water (10 mL), the layers were separated and the organic phase was washed with saturated aqueous sodium bicarbonate solution and brine. The organic phase was dried over sodium sulphate, filtered, and the filtrate was again filtered through neutral aluminium oxide (AloxN). The filtrate was then concentrated under reduced pressure and the crude product was purified by column chromatography (SiO₂, hexane/ethyl acetate) to give the title compound as a white solid.
Yield: 38.0 mg, 62 % over two steps from **12.**
¹H-NMR (300MHz, CHLOROFORM-d): δ [ppm]= 1.52 (s, 9H), 1.57 (s, 9H), 3.92 (dd, 1H), 4.24 (d, 1H), 4.31 - 4.45 (m, 2H), 4.98 (d, 1H).

### 47) Di-tert-butyl (3aS,6aS)-3a,4,6,6a-tetrahydro-3H-furo[3,4-d]-1,2,3-oxathiazole -3,3a-dicarboxylate 2,2-dioxide

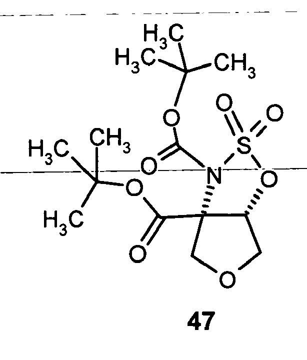

The compound was prepared according to the racemic compound **13** to give the title compound as a crystalline white solid.
¹H-NMR (300MHz, CHLOROFORM-d): δ [ppm]= 1.52 (s, 9H), 1.57 (s, 9H), 3.92 (dd, 1H), 4.24 (d, 1H), 4.31 - 4.45 (m, 2H), 4.98 (d, 1H).
Melting point: 82.8 ± 1.3°C.

### 18) tert-Butyl (3RS,4RS)-3-[(tert-butoxycarbonyl)amino]-4-{[(trifluoromethyl)sulfonyl]oxy}-tetrahydrofuran-3-carboxyalte

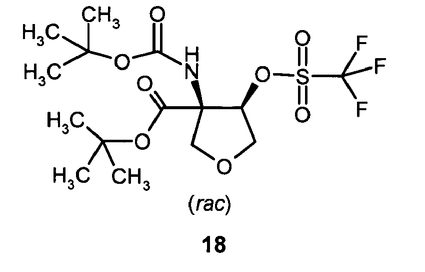

Pyridine (1.33 mL) was added to a solution of alcohol **10** (100 mg, 0.33 mmol) in dichloromethane (40 mL). After cooling to 0°C trifluoromethanesulfonic acid anhydride (0.40 mL, 2.31 mmol) was added dropwise and the mixture stirred for 15 min at this temperature. Then the mixture was concentrated at r.t. under reduced pressure and the crude product was purified by column chromatography (SiO₂, hexane/ethyl acetate) to give the title compound as a white solid.
Yield: 135 mg, 91 %.
¹H-NMR (300MHz, CHLOROFORM-d): δ [ppm]= 1.47 (s, 9H), 1.49 (s, 9H), 3.85 (d, 1H), 4.09 - 4.22 (m, 2H), 4.46 (dd, 1H), 5.06 (br. s., 1H), 5.80 (br. s., 1H).

### 19) tert-Butyl (3RS,4RS)-3-[(tert-butoxycarbonyl)amino]-4-(mesyloxy)tetrahydrofuran -3-carboxylate

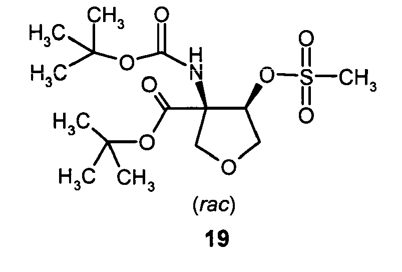

Triethylamine (0.12 mL, 0.82 mmol) and methanesulphonic acid chloride (38.3 µL, 0.49 mmol) were added to a solution of alcohol **10** (50 mg, 0.16 mmol) in dichloromethane (5 mL) at 0°C. After stirring for 2 h at this temperature the reaction mixture was diluted with ethyl acetate (20 mL) and washed with 1 N hydrochloric acid (10 mL), and brine (10 mL). The organic phase was dried over sodium sulphate and concentrated under reduced pressure. The crude product was purified by column chromatography (SiO₂, hexane/ethyl acetate) to give the title compound as a white solid.
Yield: 61 mg, 97 %.
¹H NMR (CHLOROFORM-d ,300MHz): δ = 5.47 (br. s., 1 H), 5.12 (br. s., 1 H), 4.37 (dd, *J*=10.5, 5.2 Hz, 1 H), 4.16 (d, *J*=9.4 Hz, 1 H), 4.09 (dd, *J*=10.6, 2.9 Hz, 1 H), 3.88 (d, *J*=9.4 Hz, 1 H), 3.07 (s, 3 H), 1.49 (s, 9 H), 1.46 ppm (s, 9 H).

### 20) tert-Butyl (3RS,4RS)-3-[(tert-butoxycarbonyl)amino]-4-(tosyloxy)tetrahydrofuran -3-carboxylate

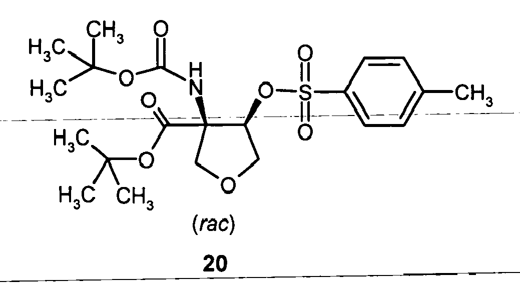

Triethylamine (0.12 mL, 0.82 mmol) and *p*-toluenesulphonic acid anhydride (167 mg, 0.51 mmol) were added to a solution of alcohol **10** (52 mg, 0.17 mmol) in dichloromethane (5 mL) at 0°C. After stirring for 2 h at this temperature the reaction mixture was diluted with ethyl acetate (20 mL) and washed with 1 N hydrochloric acid (10 mL), and brine (10 mL). The organic phase was dried over sodium sulphate and concentrated under reduced pressure. The crude product was purified by column chromatography (SiO₂, hexane/ethyl acetate) to give the title compound as a white solid.
Yield: 53 mg, 66 %.
¹H-NMR (300MHz, CHLOROFORM-d): δ [ppm]= 1.43 (br. s., 9H), 1.45 (s, 9H), 2.49 (br. s., 3H), 3.77 (dd, 1H), 3.96 - 4.12 (m, 2H), 4.31 (d, 1 H), 5.12 (t, 1H), 5.21 (br. s., 1H), 7.37 (d, 2H), 7.81 (d, 2H).

### 16) Methyl (3RS,4RS)-3-[(tert-butoxycarbonyl)amino]-4-(mesyloxy)tetrahydrofuran-3-carboxylate

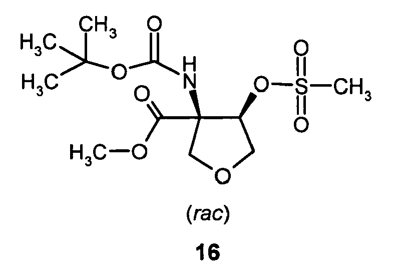

Triethylamine (0.27 mL, 1.91 mmol) and methanesulphonic acid chloride (88.9 µL, 1.15 mmol) were added to a solution of alcohol **9** (100 mg, 0.38 mmol) in dichloromethane (10 mL) at 0°C. After stirring for 2 h at this temperature the reaction mixture was diluted with ethyl acetate (20 mL) and washed with 1 N hydrochloric acid (10 mL), and brine (10 mL). The organic phase was dried over sodium sulphate and concentrated under reduced pressure. The crude product was purified by column chromatography (SiO₂, hexane/ethyl acetate) to give the title compound as a colourless oil.
Yield: 120 mg, 88 %.
¹H-NMR (300MHz, CHLOROFORM-d): δ [ppm]= 1.45 (s, 9H), 3.08 (s, 3H), 3.82 (s, 3H), 3.91 (d, 1H), 4.09 (dd, 1H), 4.20 (d, 1H), 4.38 (dd, 1H), 5.18 (br. s., 1H), 5.51 (dd, 1H).

### 17) Methyl (3RS,4RS)-3-[(tert-butoxycarbonyl)amino]-4-(tosyloxy)tetrahydrofuran -3-carboxylate

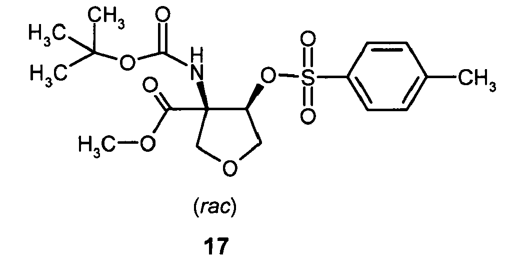

Triethylamine (0.27 mL, 1.91 mmol) and *p*-toluenesulphonic acid anhydride (374 mg, 1.15 mmol) were added to a solution of alcohol **9** (100 mg, 0.38 mmol) in dichloromethane (10 mL) at 0°C. After stirring for 2 h at this temperature the reaction mixture was diluted with ethyl acetate (20 mL) and washed with 1 N hydrochloric acid (10 mL), and brine (10 mL). The organic phase was dried over sodium sulphate and concentrated under reduced pressure. The crude product was purified by column chromatography (SiO₂, hexane/ethyl acetate) to give the title compound as a colourless oil.
Yield: 145 mg, 91 %.
¹H-NMR (300MHz, CHLOROFORM-d): δ [ppm]= 1.43 (s, 9H), 2.47 (s, 3H), 3.71 (s, 3H), 3.81 (dd, 1H), 3.95 - 4.18 (m, 2H), 4.31 (d, 1H), 5.10 - 5.29 (m, 2H), 7.38 (d, 2H), 7.80 (d, 2H).

### 14) N-(tert-Butoxycarbonyl)-N-[(3SR,4SR)-3-(tert-butoxycarbonyl)-4-fluorotetrahydrofuran -3-yl]sulfamic acid

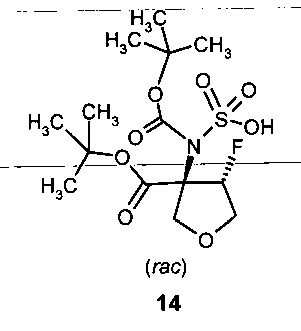

Tetrabutylammonium fluoride (1.0 M in THF, 0.27 mL, 0.27 mmol) was added to a solution of sulphamidate **13** (50.0 mg, 0.14 mmol) in acetonitrile (2 mL) and the mixture was heated to 50°C for 40 min under microwave irradiation. After that more tetrabutylammonium fluoride (1.0 M in THF, 0.27 mL, 0.27 mmol) was added and the mixture was heated to 60°C for 20 min under microwave irradiation. Then the mixture was concentrated under reduced pressure and directly used in the next step.
MS (ESneg): m/z = 384 [M-H]⁻.

### 15) (3SR,4SR)-3-Amino-4-fluorotetrahydrofuran-3-carboxylic acid

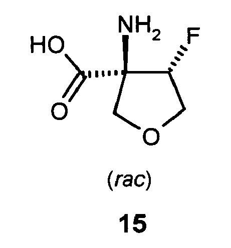

The crude sulphamic acid **14** was taken up in 4 N aqueous hydrochloric acid (1 mL) and the mixture was heated to 50°C for 10 min. After that the mixture was concentrated under reduced pressure and the crude product purified by preparative HPLC (XBrigde C18 5µm 150x19 mm, 0.1% aqueous TFA/acetonitrile gradient, 21 mL/min).
Yield: 20 mg, 88% over two steps from **13.**
¹H-NMR (400MHz, DMSO-d₆): δ [ppm]= 3.86 (d, 1H), 3.91 - 4.04 (m, 1H), 4.11 - 4.25 (m, 1H), 4.36 (d, 1H), 5.33 (d, 1H), 7.02 - 7.43 (m, 1H), 9.06 (br. s., 2H).
¹⁹F-NMR (376MHz, DMSO-d₆): δ [ppm]= -185.12 (m).
MS (ESIpos): m/z = 150 [M+H]⁺.

### 51) (3R,4R)-3-Carboxy-4-fluorotetrahydrofuran-3-ammonium formate

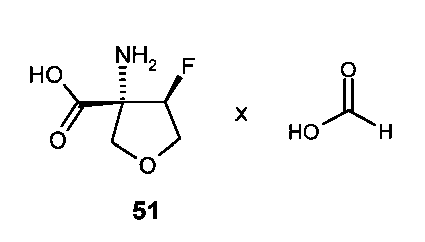

This compound was synthesized according to **15** starting from enantiopure sulphamidate precursor **47.** The crude product purified by two preparative HPLCs (1.: XBrigde C18 5µm 100x30 mm, 0.1% aqueous TFA/acetonitrile gradient, 42 mL/min; 2.: ZIC-HILIC 5µm 250x10 mm, 0.1 M aqueous ammonium formate/acetonitrile gradient, 10 mL/min).
Yield: 12 mg, 22% over two steps from **47).**
¹H-NMR (400MHz, DEUTERIUM OXIDE): δ [ppm]= 3.93 (d, 1H), 4.02 (ddd, 1H), 4.23 (ddd, 1H), 4.39 (d, 1H), 5.28 (ddd, 1H), 8.39 (s, 1H).
¹⁹F-NMR (376MHz, DEUTERIUM OXIDE): δ [ppm]= -185.82 (m).

### Example 3

### 22) (SR)-4,4-Dimethoxy-3,4,5,6-tetrahydro-2H-pyran-3-ol

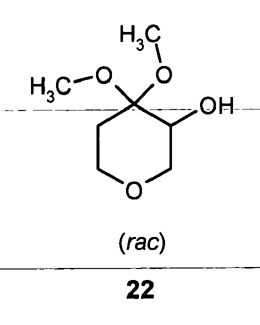

Tetrahydropyran-4-one (**21**) (1.52 g, 19.2 mmol) and potassium hydroxide (2.40 g, 85 %, 36.4 mmol) were dissolved in methanol (30 mL) under a nitrogen atmosphere. The resulting solution was cooled to 0 °C and a solution of iodine (4.24 g, 16.8 mmol) in methanol (35 mL) was added dropwise over a period of 2 h. Afterwards the reaction mixture was allowed to warm up to room temperature and stirred for 1 h at this temperature. Then the solvent was removed under reduced pressure and the residue was suspended in toluene (40 mL). After filtration, the solvent was removed under reduced pressure to give the crude product which was used without further purification in the next step.
Yield: 2.08 g, 84 %.

### 23) (SR)-3-Benzyloxy-4,4-dimethoxy-3,4,5,6-tetrahydro-2H-pyran

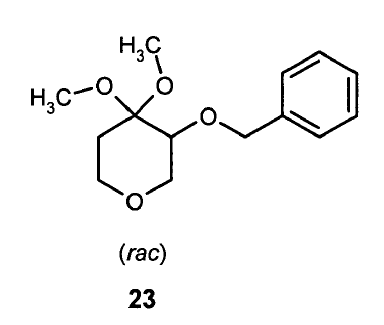

A solution of 4,4-dimethoxy-tetrahydropyran-3-ol (**22**) (850 mg, 5.24 mmol) in THF (20 mL) under nitrogen atmosphere was cooled down to 0 °C before sodium hydride (202 mg, 60 % on mineral oil, 5.03 mmol) was added in one portion. The resulting suspension was stirred at this temperature for 30 min. Afterwards benzylbromide (0.6 mL, 861 mg, 5.03 mmol) and a catalytic amount of tetrabutyl ammonium iodide were added and the resulting mixture was allowed to warm up to room temperature. After stirring for 20 h a saturated aqueous ammonium chloride solution (15 mL) was added, the phases were separated and the aqueous phase was extracted with diethylether (3 x 15 mL). The combined organic extracts we washed with water and brine, dried over magnesium sulphate, filtrated and the solvent was removed under reduced pressure to give the title compound which was directly used in the next step.
Yield: 1.16g,88%.

### 24) (SR)-3-Benzyloxy-3,4,5,6-tetrahydro-2H-pyran-4-one

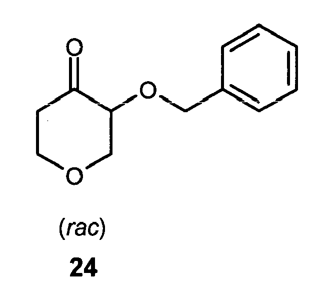

3-Benzyloxy-4,4-dimethoxy-tetrahydropyran (**23**) (545 mg, 2.16 mmol) was dissolved in a TFA/H₂O/CHCl₃ mixture (1:1:4, 3 mL) and heated to 50°C. The resulting solution biphasic mixture was stirred for 2.5 h at this temperature and then cooled to room temperature. Then the mixture was neutralized by addition of a saturated aqueous sodium carbonate solution, diethylether was added and the phases were separated. The aqueous phase was extracted with diethylether (3 x 10 mL) and the combined organic layer was washed with saturated aqueous sodium carbonate solution, water and brine, dried over magnesium sulphate, filtrated and the solvent was removed under reduced pressure to give the desired ketone.
Yield: 430 mg, 96 %.

### 25) (5RS,6RS)-6-(Benzyloxy)-8-oxa-1,3-diazaspiro[4.5]decane-2,4-dione

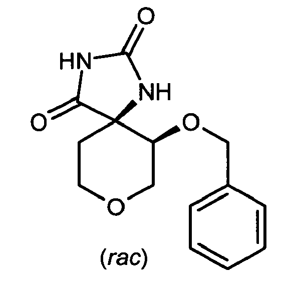

To a solution of 3-benzyloxy-tetrahydropyran-4-one (**24**) (430 mg, 2.08 mmol) in methanol (20 mL) were added ammonium carbonate (2.00 g, 20.8 mmol) and ammonium chloride (446 mg, 8.34 mmol) in water (20mL). The resulting mixture was stirred for 30 min at room temperature, before potassium cyanide (611 mg, 9.4 mmol) was added and the reaction mixture was heated to 60 °C. After stirring for 18 h at this temperature, the reaction was allowed to cool down to room temperature. After addition of water (20 mL) and ethyl acetate (30 mL) the phases were separated and the aqueous phase was extracted ethyl acetate (3x 30 mL). The combined organic layer was dried over magnesium sulphate, filtrated and the solvent was removed under reduced pressure. The crude product was purified by column chromatography (SiO₂, hexane/ethyl acetate gradient) to give the title compounds as a diastereomeric mixture of ratios from 75:25-90:10 in favour of the shown (5*RS*,6*RS*)-diastereomer **25.**
Yield: 303 mg, 53 %.
¹H-NMR (400MHz, DMSO-d₆): δ [ppm]= 1.64 (d, 1H), 1.88 (td, 1H), 3.32 - 3.49 (m, 2H), 3.64 (dd, 1H), 3.69 - 3.77 (m, 1H), 3.88 (dd, 1H), 4.44 (d, 1H), 4.55 (d, 1H), 7.20 - 7.36 (m, 5H), 8.53 (s, 1H), 10.76 (br. s., 1H).
MS (ESIpos): m/z = 277 [M+H]⁺

### 28) tert-Butyl (3RS,4RS)-3-benzyloxy-4-[(tert-butoxycarbonyl)amino]-3,4,5,6-tetrahydro-2H-pyran-4-carboxylate

### 29) tert-Butyl (3SR,4RS)-3-benzyloxy-4-[(tert-butoxycarbonyl)amino]-3,4,5,6-tetrahydro-2H-pyran-4-carboxylate

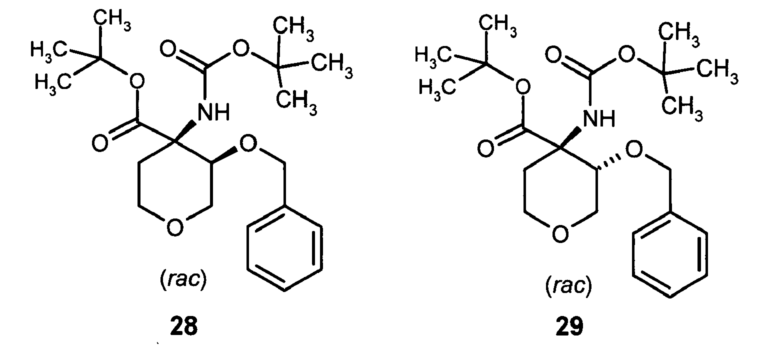

A solution of hydantoin **25** (20.5 g, 74.2 mmol) (present as the above described diastereomeric mixture) in 3 M aqueous sodium hydroxide solution (250 mL) was stirred under reflux for 8 days. Then more 3M aqueous sodium hydroxide solution (600mL) was added and the reaction mixture was stirred under reflux for 4 days. After cooling to r.t. the pH of the mixture was adjusted to 6-7 with concentrated hydrochloric acid and then lyophilised.

The crude amino acid **26** was suspended in methanol/triethylamine (9:1, 1.5 L) at r.t. and di-tert-butyl-dicarbonate (97.1 g, 445 mmol) was added. After stirring for 1 day more triethylamine (50 mL) and more di-tert-butyl-dicarbonate (46.0 g, 211 mmol) were added. After 3 day more di-tert-butyl-dicarbonate (46.0 g, 211 mmol) was added and the mixture stirred for further 2 days. Then concentrated aqueous sodium hydroxide solution (200 mL) was added and the mixture stirred for 20 min at r.t.. After that the pH was adjusted to 6-7 with concentrated hydrochloric acid and the mixture was concentrated under reduced pressure to remove the methanol and then extracted with ethyl acetate (3 x 500 mL). The combined organic layers was washed with 1 M aqueous potassium hydrogen sulphate solution (1 x 500 mL) and then concentrated under reduced pressure.
The crude *N-*Boc-protected amino acid **27** was dissolved in dichloromethane (1 L) at r.t. and tert-butyl 2,2,2-trichloroacetimidate (40.8 g, 187 mmol) was added. After 5 days more tert-butyl 2,2,2-trichloroacetimidate (20.4 g, 93.3 mmol) was added and the mixture stirred again for 6 days. Then the mixture was filtered and the filtrate was concentrated under reduced pressure. The crude product was purified by column chromatography (SiO₂, hexane/ethyl acetate gradient) to give **28** and **29.**

### 28):

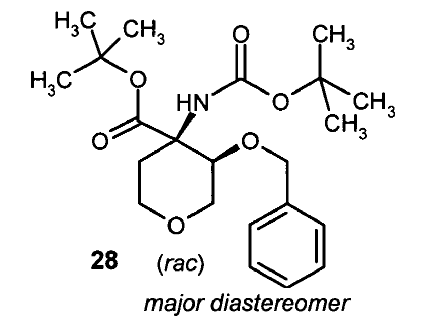

Yield: 11.6 g, 61 %.
¹H-NMR (300MHz, CHLOROFORM-d): δ [ppm]= 1.47 (s, 9H), 1.49 (s, 9H), 2.02 - 2.17 (m,
1H), 2.84 (d, 1H), 3.42 - 3.63 (m, 2H), 3.68 - 3.92 (m, 3H), 4.47 - 4.62 (m, 2H), 5.13 (br. s., 1H), 7.26 -7.41 (m, 5H).
MS (ESIpos): m/z = 394 [M+H]⁺

### 29):

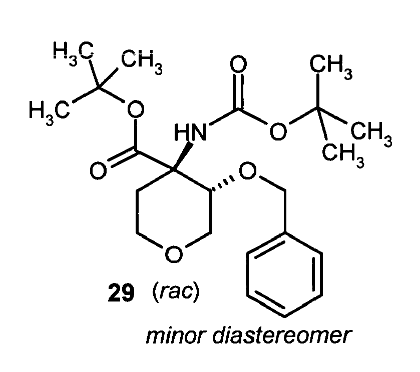

Yield: 380 mg, 6 %.
¹H-NMR (400MHz, CHLOROFORM-d): δ [ppm]= 1.44 (s, 9H), 1.47 (s, 9H), 2.25 (d, 1H), 2.34 - 2.44 (m, 1H), 3.62 - 3.74 (m, 3H), 3.79 - 3.87 (m, 1H), 3.87 - 3.96 (m, 1H), 4.56 (d, 1H), 4.66 (d, 1H), 5.01 (br. s., 1H), 7.28 - 7.34 (m, 5H).
MS (ESIpos): m/z = 394 [M+H]⁺

### 30) tert-Butyl (3RS,4RS)-4-[(tert-butoxycarbonyl)amino]-3-hydroxy-3,4,5,6-tetrahydro-2H-pyran-4-carboxylate

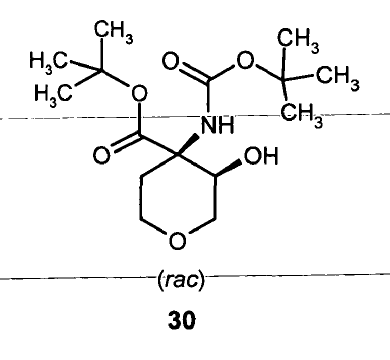

A mixture of **28** (49.6 mg, 0.12 mmol) and palladium (5 mg, 10 % on charcoal) in methanol (6 mL) was stirred for 14 h at r.t. under a hydrogen atmosphere. Then the mixture was filtered over Celite, the filter cake rinsed thoroughly with methanol and the filtrate was concentrated under reduced pressure.
Yield: 36.8 g, 95 %.
¹H-NMR (300MHz, CHLOROFORM-d): δ [ppm]= 1.43 - 1.48 (m, 9H), 1.50 (s, 9H), 2.08 - 2.30 (m, 2H), 3.19 (br. s., 1H), 3.47 (dd, 1H), 3.56 - 3.68 (m, 1H), 3.79 (td, 2H), 3.90 - 4.03 (m, 1H), 5.18 (s, 1H).
MS (ESIpos): m/z = 318 [M+H]⁺.

### 31) tert-Butyl (3SR,4RS)-4-[(tert-butoxycarbonyl)amino]-3-hydroxy-3,4,5,6-tetrahydro-2H-pyran-4-carboxylate

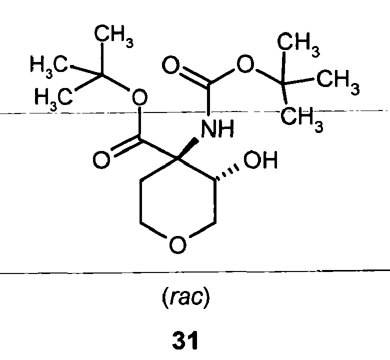

A mixture of **29** (24.0 mg, 58.9 µmol) and palladium (10 mg, 10 % on charcoal) in methanol (2 mL) was stirred for 14 h at r.t. under a hydrogen atmosphere. Then the mixture was filtered over Celite, the filter cake rinsed thoroughly with methanol and the filtrate was concentrated under reduced pressure.
Yield: 5.0 mg, 24 %.
¹H-NMR (400MHz, CHLOROFORM-d): □ [ppm]= 1.46 (s, 9H), 1.50 (s, 9H), 2.10 - 2.27 (m, 2H), 3.20 (br. s., 1H), 3.47 (dd, 1H), 3.57 - 3.69 (m, 1H), 3.73 - 3.85 (m, 2H), 3.92 - 4.02 (m, 1H), 5.18 (br. s., 1H).
MS (ESIpos): m/z = 318 [M+H]⁺.

### 32) Di-tert-butyl (3aRS,7aRS)-3,3a,4,5,7,7a-hexahydropyrano[4,3-d]-1,2,3-oxathiazole-3,3a-dicarboxylate 2,2-dioxide

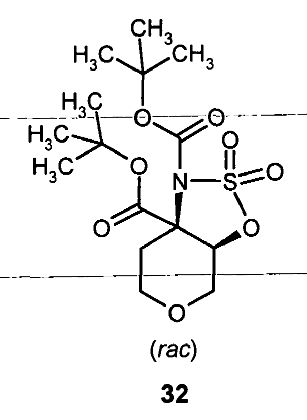

Sodium hydride (60 % on mineral oil, 5.8 mg) was added at r.t. to a solution of alcohol **30** (770 mg, 2.43 mmol) in THF (15 mL). After that a solution of sulphonyl diimidazolide (625 mg, 3.15 mmol) in THF (5 mL) was added dropwise at r.t. to the mixture. After stirring for 1 h at this temperature the reaction was quenched by addition of saturated aqueous sodium bicarbonate solution. The mixture was extracted with ethyl acetate and the organic layer was concentrated under reduced pressure. The crude product was purified by column chromatography (SiO₂, hexane/ethyl acetate) to give the title compound as a white solid.
Yield: 640 mg, 70 %.
¹H-NMR (400MHz, CHLOROFORM-d): δ [ppm]= 1.51 (s, 9H), 1.56 (s, 9H), 2.21 (ddd, 1H), 2.71 (d, 1H), 3.65 - 3.77 (m, 2H), 3.93 - 4.02 (m, 1H), 4.28 (d, 1H), 4.68 (s, 1H).
MS (ESIpos): m/z = 397 [M+H₂O]⁺.
Melting point: 102.3 °C.

### 34) tert-Butyl (3RS,4RS)-4-[(tert-butoxycarbonyl)amino]-3-(tosyloxy)-3,4,5,6-tetrahydro-2H-pyran-4-carboxylate

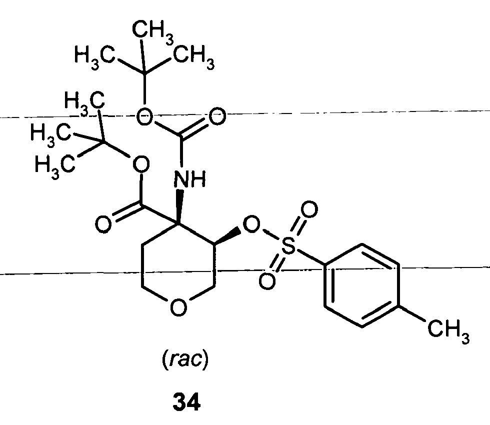

To a solution of alcohol **30** (110 mg, 0.35 mmol) in dry dichloromethane (10 mL) was added at 0°C triethylamine (0.24 mL, 1.73 mmol) and p-toluenesulphonic anhydride (339 mg, 1.04 mmol). The mixture was stirred for 14 h at r.t. and then diluted with ethyl acetate and washed with 1 N hydrochloric acid and brine. After drying over sodium sulphate the solvent was removed under reduced pressure and the crude product was purified by column chromatography (SiO₂ hexane/ethyl acetate) to give the product as a white solid.
Yield: 71 mg, 43 %.
¹H-NMR (400MHz, CHLOROFORM-d): δ [ppm]= 1.50 (d, 18H), 2.07 (ddd, 1H), 2.52 (s, 4H), 3.48 - 3.81 (m, 4H), 4.83 - 5.01 (m, 2H), 7.36 (s, 2H), 7.81 (d, 2H).
MS (ESIneg): m/z = 470 [M-H]⁻.

### 33) tert-Butyl (3RS,4RS)-4-[(tert-butoxycarbonyl)amino]-3-(mesyloxy)-3,4,5,6-tetrahydro-2H-pyran-4-carboxylate

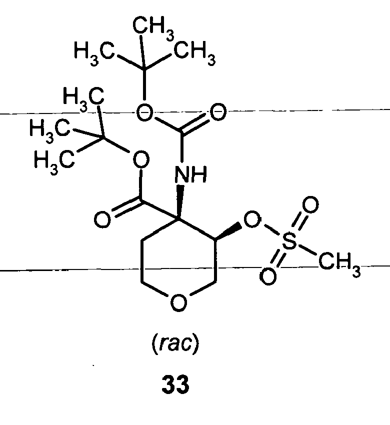

To a solution of alcohol **30** (50 mg, 0.16 mmol) in dry dichloromethane (5 mL) was added at 0°C triethylamine (0.11 mL, 0.79 mmol) and methanesulphonic acid chloride (36.6 µL, 0.47 mmol). The mixture was stirred for 14 h at r.t. and then diluted with ethyl acetate and washed with 1 N hydrochloric acid and brine. After drying over sodium sulphate the solvent was removed under reduced pressure and the crude product was purified by column chromatography (SiO₂ hexane/ethyl acetate) to give the product as a white solid.
Yield: 54.5 mg, 87 %.
¹H-NMR (300MHz, CHLOROFORM-d): d [ppm]= 1.46 (s, 9H), 1.50 (s, 9H), 1.94 - 2.11 (m, 1H), 2.41 (br. s., 1H), 3.05 (s, 3H), 3.55 - 3.67 (m, 1H), 3.70 - 3.81 (m, 1H), 3.87 - 3.99 (m, 1H), 4.06 - 4.16 (m, 1H), 4.88 (br. s., 1H), 5.09 (dd, 1H).
MS (ESIneg): m/z = 394 [M-H]⁻.

### Example 4: General Radiolabeling of Sulfonate

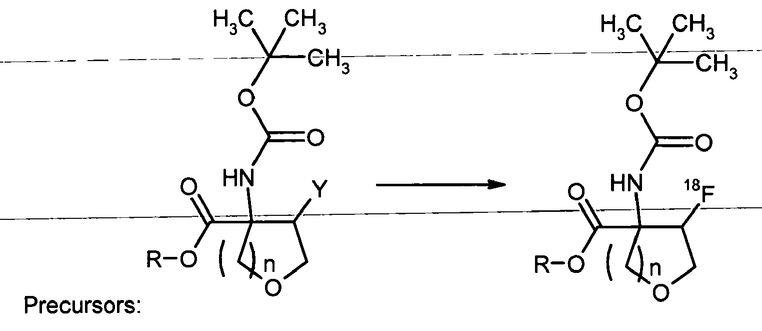

[¹⁸F]Fluoride was immobilized on a preconditioned QMA cartridge (preconditioned by washing the cartridge with 5 mi 0.5 M K₂CO₃ and 10 ml water), The [F-18]fluoride was eluted using either of the following solutions:
i) K₂CO₃ (1 mg) in 500 µl water and K222 (5 mg) in 1500 µl acetonitrile
ii) Cs₂CO₃ (2,3 mg) in 500 µl water and K222 (5 mg) in 1500 µl acetonitrile
iii) TBAOH (8 µl 40% w/v in water) in 500 µl water in 1500 µl acetonitrile

This eluted solution was dried at 120°C with stirring with a stream of nitrogen. Additional acetonitrile (1 ml) was added and the drying under a nitrogen stream repeated. A solution of 2 mg precursor in acetonitrile (300µl) was added and heated at 100°C for 10 mins. The incorporation of fluoride was determined by HPLC and shown in the table below (HPLC Column: ACE 3µ C18 50 x 4,6 mm, Solvent A: 10 mM K₂HPO₄ in water, Solvent B: 10 mM K₂HPO₄ in water/acetonitrile (7:3), Gradient: 5% B to 95% B in 7mins, 95% B to 100% B in 6 secs, 100% B for 102 secs, 100% B to 5% B in 12 secs, 5% B for 3 mins, Flow: 2ml/min, detection: radioactive.

**Table 1. Radiofluorination of Sulfonate Precursors**

| **Sulphonate** | **n** | **Y** | **R** | **Base** | **Amount K222 (mg)** | **Incorporation (%)** |
|---|---|---|---|---|---|---|
| **17** | 1 | Tosylate | Me | CS₂CO₃ | 5 | 5 |
| **18** | 1 | Triflate | tBu | CS₂CO₃ | 5 | 4.8 |
| **18** | 1 | Triflate | tBu | K₂CO₃ | 5 | 18.4 |
| **18** | 1 | Triflate | tBu | TBAOH | 0 | 12.6 |
| **34** | 2 | Tosylate | tBu | CS₂CO₃ | 5 | 17.6 |
| **34** | 2 | Tosylate | tBu | K₂CO₃ | 5 | 17.8 |
| **34** | 2 | Tosylate | tBu | TBAOH | 0 | 5.6 |

### Example 5:

### 45) (3SR,4SR)-3-Am ino-4-[¹⁸F]fluorotetrahydrofuran-3-carboxylic acid

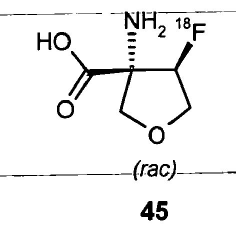

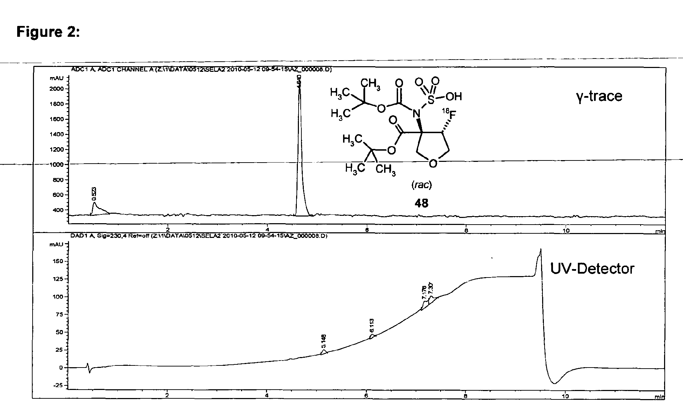

[¹⁸F]Fluoride (4287 MBq) was immobilized on a preconditioned QMA cartridge (preconditioned by washing the cartridge with 5 ml 0.5 M K₂CO₃ and 10 ml water), The [F-18]fluoride was eluted using a solution of K₂CO₃ (1 mg) in 50 µl water and K222 (5 mg) in 950 µl acetonitrile. This solution was dried at 120°C with stirring with a stream of nitrogen. Additional acetonitrile (1 ml) was added and the drying under a nitrogen stream repeated. A solution of **13** (2 mg) in acetonitrile (300 µl) was added and heated at 120°C for 15 min for obtaining intermediate **48.** HPLC analysis of the intermediate **48** was carried out (HPLC Column: ACE 3µ C18 50 * 4,6 mm, Solvent A: Water + 0.1%TFA, Solvent B: Acetonitrile + 0.1%TFA, Gradient: 5% B to 95% B in 7 min, 95% B to 100% B in 6 sec, 100% B for 102 sec, 100% B to 5% B in 12 sec and 5% B for 3 min, Flow: 2 ml/min), figure 2.
The reaction mixture was diluted with acetonitrile (1 ml) and passed through a silica Plus SPE (preconditioned with 10 ml acetonitrile). The SPE was washed with acetonitrile (2 x 1 ml). HPLC analysis of the purified intermediate **48** was carried out (HPLC Column: ACE 3µ C18 50 * 4,6 mm, Solvent A: Water + 0.1%TFA, Solvent B: Acetonitrile + 0.1%TFA, Gradient: 5% B to 95% B in 7 min, 95% B to 100% B in 6 sec, 100% B for 102 sec, 100% B to 5% B in 12 sec and 5% B for 3 min, Flow: 2 ml/min).
The acetonitrile elutions were combined and concentrated to dryness at 70°C under a stream of nitrogen. To the dried reaction mixture was added 500µl 6M HCl and the mixture was incubated for 20min at 120°C. After cooling to 60°C, the reaction mixture was diluted with 4ml water and passed through a series of cartridges i) AG11A8 resin (ion retardation resin, ∼11g); ii) Alumina N Plus SPE (preconditioned with 10ml water); iii) HLB SepPak (preconditioned with 5ml ethanol and with 10ml water); all three cartridges were then washed with 60ml water]. The desired product, 1185 MBq (54,9% d.c.), was eluted from the cartridges with water (10ml) in an unoptimized synthesis time of 109 min. HPLC analysis of the product was confirmed by co-injection (HPLC Column: ZIC-HILIC 5µm 200A, 100 x 4.6mm (SeQuant), Solvent A: 0.1M ammonium formate in water pH 3.2, Solvent B: Acetonitrile, Gradient: 80%B for 5 min, 80% B to 10% B in 1 min, 10% B for 2 min, 10% B to 80% B in 1 min and 80% B for 2 min, Flow: 2 ml/min, detection: Corona CAD (Elisa)).

Figure 2 shows the chromatogram of intermediate **48** obtained from incorporation of [¹⁸F]fluoride from precursor **13** (Example 5)

| | | |
|---|---|---|
| HPLC-System: | Agilent 1100 | |
| | | |
| HPLC Column: | ACE 3µ C18 50*4,6 mm | |
| Eluents: | A: Water + 0.1 % TFA (Agilent 7) | |
| | B: Acetonitrile + 0.1 % TFA | |
| | | |
| Gradient | 00:00 min | 05%B |
| | 07:00 | 95%B |
| | 07:10 | 100%B |
| | 08:80 | 100%B |
| | 09:00 | 05%B |
| | 12:00 | 05%B |
| | | |
| Flow: | 2ml/min | |

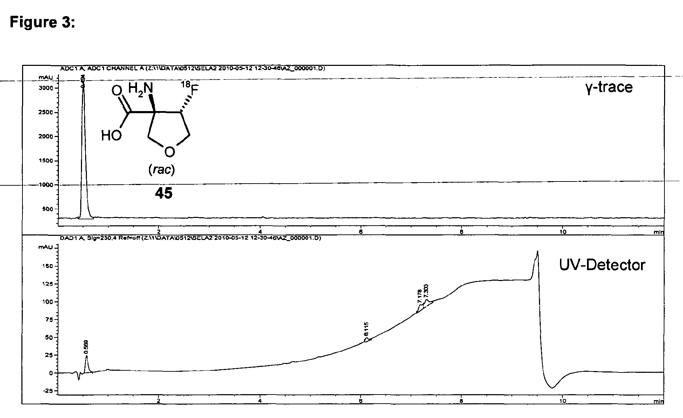

Figure 3 shows the chromatogram of th final product **45** after acidic deprotection (C18 HPLC column)

| | | |
|---|---|---|
| HPLC System: | Agilent 1100 | |
| HPLC Column: | ACE 3µ C18 50 * 4,6 mm | |
| Eluents: | A: Water + 0.1 % TFA (Agilent 7) | |
| | B: Acetonitrile + 0.1 % TFA | |
| | | |
| Gradient | 00:00 min | 05%B |
| | 07:00 | 95%B |
| | 07:10 | 100%B |
| | 08:80 | 100%B |
| | 09:00 | 05%B |
| | 12:00 | 05%B |
| | | |
| Flow: | 2 ml/min | |

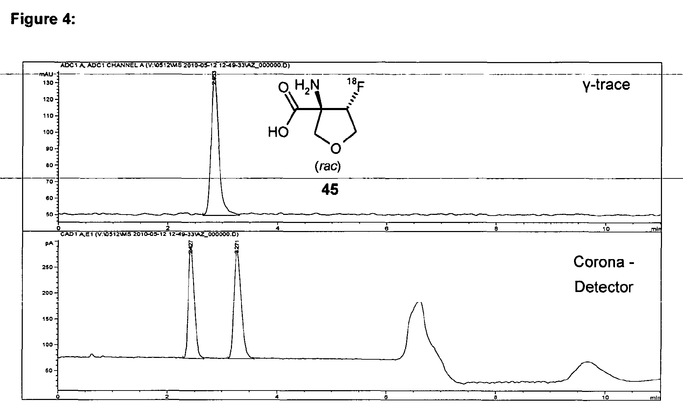

Figure 4 shows the chromatogram of the final product **45** after acidic deprotection (HILIC HPLC column with CAD Corona Detector)

| | | |
|---|---|---|
| HPLC System: | Agilent 1100 | |
| | | |
| HPLC Column: | ZIC HILIC 5µ 200A 100*4,6mm | |
| Eluents: | A: 0.1 M Ammonium formiate pH3,2 (Agilent 5) | |
| | B: Acetonitrile | |
| Gradient: | 00:00 min | 80%B |
| | 05:00 | 80%B |
| | 06:00 | 10%B |
| | 08:00 | 10%B |
| | 09:00 | 80%B |
| | 11:00 | 80%B |
| Flow: | 2 ml/min | |

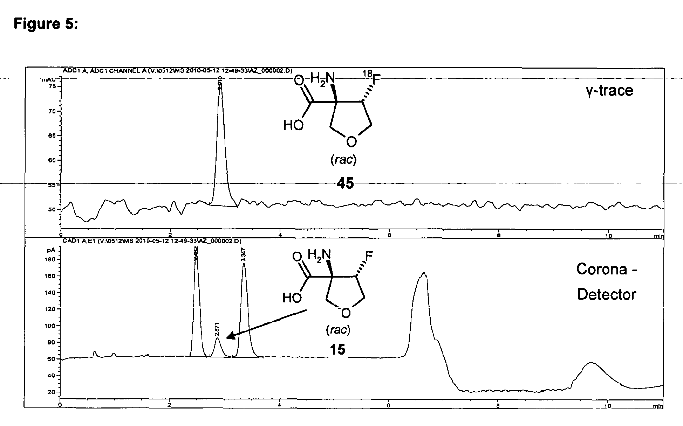

Figure 5 shows chromatogram of the final product **45** after acidic deprotection co-injected with the cold standard **15** (HILIC HPLC column with CAD Corona Detector)

| | | |
|---|---|---|
| HPLC System: | Agilent 1100 | |
| | | |
| HPLC Column: | ZIC HILIC 5µ 200A 100*4,6mm | |
| Eluents: | A: 0.1 M Ammonium formiate pH3,2 (Agilent 5) | |
| | B: Acetonitrile | |
| | | |
| Gradient: | 00:00 min | 80%B |
| | 05:00 | 80%B |
| | 06:00 | 10%B |
| | 08:00 | 10%B |
| | 09:00 | 80%B |
| | 11:00 | 80%B |
| | | |
| Flow: | 2 ml/min | |

### Example 6:

### 51) (3R,4R)-3-Amino-4-[¹⁸F]fluorotetrahydrofuran-3-carboxylic acid

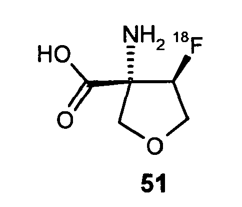

[¹⁸F]Fluoride (2117 MBq) was immobilized on a preconditioned QMA cartridge (preconditioned by washing the cartridge with 5 ml 0.5 M K₂CO₃ and 10 ml water), The [F-18]fluoride was eluted using a solution of K₂CO₃ (1 mg) in 50 µl water and K222 (5 mg) in 950 µl acetonitrile. This solution was dried at 120°C with stirring with a stream of nitrogen. Additional acetonitrile (1 ml) was added and the drying under a nitrogen stream repeated. A solution of **47** (2 mg) in acetonitrile (300 µl) was added and heated at 120°C for 15 min. The reaction mixture was diluted with acetonitrile (1 ml) and passed through a silica Plus SPE (preconditioned with 10 ml acetonitrile). The SPE was washed with acetonitrile (2 x 1 ml). The acetontrile elutions were combined and concentrated to dryness at 70°C under a stream of nitrogen. To the dried reaction mixture was added 500 µl 6M HCl and the mixture was incubated for 20 min at 120°C. After cooling to 60°C, the reaction mixture was diluted with 4ml water and passed through a series of cartridges [i) AG11A8 resin (ion retardation resin, ∼11 g); ii) Alumina N Plus SPE (preconditioned with 10ml water); iii) HLB SepPak (preconditioned with 5ml ethanol and with 10ml water); all three cartridges were then washed with 60ml water]. The desired product, 721 MBq (63% d.c.), was eluted from the cartridges with water (10ml) in an unoptimized synthesis time of 98 min. HPLC analysis of the product was confirmed by co-injection (HPLC Column: ZIC-HILIC 5µm 200A, 100 x 4.6mm (SeQuant), Solvent A: 0.1M ammonium formate in water pH 3.2, Solvent B: Acetonitrile, Gradient: 80%B for 5mins, 80% B to 10% B in 1 min, 10% B for 2 mins, 10% B to 80% B in 1 min and 80% B for 2 min, Flow: 2ml/min, detection: Corona CAD (Elisa)).

### Example 7:

### 46) (3RS,4RS)-4-Amino-3-[¹⁸F]fluoro-3,4,5,6-tetrahydro-2H-pyran-4-carboxylic acid

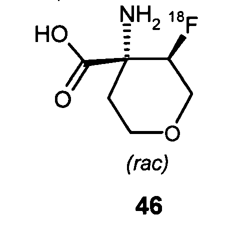

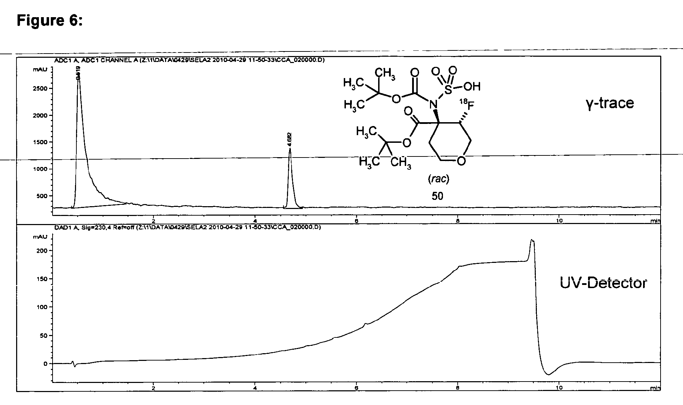

[¹⁸F]Fluoride (3111 MBq) was immobilized on a preconditioned QMA cartridge (preconditioned by washing the cartridge with 5 ml 0.5 M K₂CO₃ and 10 ml water), The [F-18]fluoride was eluted using a solution of K₂CO₃ (1 mg) in 50 µl water and K222 (5 mg) in 950 µl acetonitrile. This solution was dried at 120°C with stirring with a stream of nitrogen. Additional acetonitrile (1 ml) was added and the drying under a nitrogen stream repeated. A solution of **32** (2 mg) in acetonitrile (300 µl) was added and heated at 120°C for 15 min for obtaining intermediate **50.** HPLC analysis of the intermediate **50** was carried out (HPLC Column: ACE 3µ C18 50 * 4,6 mm Eluents A: Water + 0.1% TFA (Agilent 7) Solvent B: Acetonitrile + 0.1% TFA, Gradient 00:00 min 05%B, 07:00 min 95%B, 07:10min 100%B, 08:80 min 100%B, 09:00 min05%B, 12:00 min 05%B, Flow: 2ml/min, see figure 6. The reaction mixture was diluted with acetonitrile (1 ml) and passed through a silica Plus SPE (preconditioned with 10 ml acetonitrile). The SPE was washed with acetonitrile (2 x 1 ml). HPLC analysis of the purified intermediate **50** was carried out see figure 7. The acetontrile elutions were combined and concentrated to dryness at 70°C under a stream of nitrogen. To the dried reaction mixture was added 500 µl 6 M HCl and the mixture was incubated for 20min at 120°C. After cooling to 60°C, the reaction mixture was diluted with 4ml water and passed through a series of cartridges [i) AG11A8 resin (ion retardation resin, ∼11 g); ii) Alumina N Plus SPE (preconditioned with 10ml water); iii) HLB SepPak (preconditioned with 5 ml ethanol and with 10ml water); all three cartridges were then washed with 60ml water]. The desired product, 621 MBq (37% d.c.), was eluted from the cartridges with water (10ml) in an unoptimized synthesis time of 99 min. HPLC analysis using HPLC Column: ZIC-HILIC 5µm 200A, 100 x 4.6mm (SeQuant), Solvent A: 0.1M ammonium formate in water pH 3.2, Solvent B: Acetonitrile, Gradient: 80%B for 5mins, 80% B to 10% B in 1 min, 10% B for 2 mins, 10% B to 80% B in 1 min and 80% B for 2 min, Flow: 2ml/min, detection: Corona CAD (Elisa).

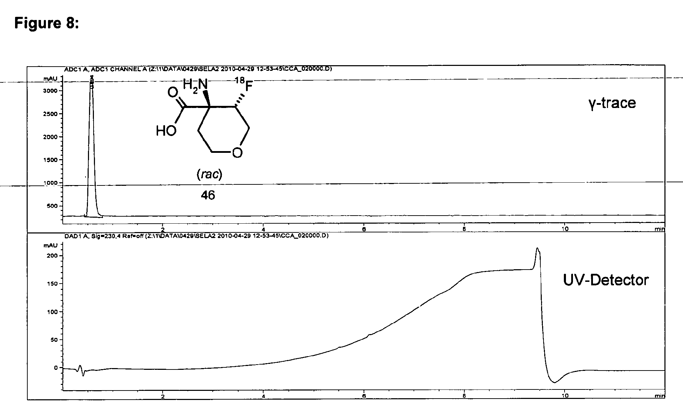

Figure 8 shows a chromatogram of one enantiomer of the final product **46** after acidic deprotection (C18 HPLC column).

| | | |
|---|---|---|
| HPLC System: | Agilent 1100 | |
| HPLC Column: | ACE 3µ C18 50 * 4,6 mm | |
| Eluents: | A: Water + 0.1 % TFA (Agilent 7) | |
| | B: Acetonitrile + 0.1 % TFA | |
| | | |
| Gradient | 00:00 min | 05%B |
| | 07:00 | 95%B |
| | 07:10 | 100%B |
| | 08:80 | 100%B |
| | 09:00 | 05%B |
| | 12:00 | 05%B |
| | | |
| Flow: | 2 ml/min | |

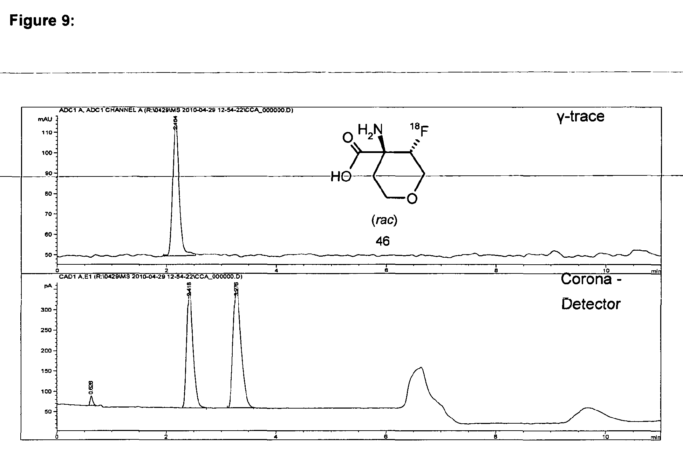

Figure 9 shows the chromatogram of one enantiomer of the final product **46** after acidic deprotection (HILIC HPLC column with CAD Corona Detector).

| | | |
|---|---|---|
| HPLC System: | Agilent 1100 | |
| HPLC Column: | ZIC HILIC 5µ 200A 100*4,6mm | |
| Eluents: | A: 0.1 M Ammonium formiate pH3,2 (Agilent 5) | |
| | B: Acetonitrile | |
| | | |
| Gradient: | 00:00 min | 80%B |
| | 05:00 | 80%B |
| | 06:00 | 10%B |
| | 08:00 | 10%B |
| | 09:00 | 80%B |
| | 11:00 | 80%B |
| | | |
| Flow: | 2 ml/min | |

Figure 6 shows chromatogram of radiofluorinated intermediate **50** obtained from incorporation of [¹⁸F]fluoride from precursor **32** (Example 7).

| | | |
|---|---|---|
| HPLC System: | Agilent 1100 | |
| | | |
| HPLC Column: | ACE 3µ C18 50 * 4,6 mm | |
| Eluents: | A: Water + 0.1 % TFA (Agilent 7) | |
| | B: Acetonitrile + 0.1% TFA | |
| | | |
| Gradient | 00:00 min | 05%B |
| | 07:00 | 95%B |
| | 07:10 | 100%B |
| | 08:80 | 100%B |
| | 09:00 | 05%B |
| | 12:00 | 05%B |
| | | |
| Flow: | 2ml/min | |

Figure 7 shows the chromatogram of radiofluorinated intermediate **50** purified by SPE from precursor **32** (Example 7).

| | | |
|---|---|---|
| HPLC System: | Agilent 1100 | |
| | | |
| HPLC Column: | ACE 3µ C18 50 * 4,6 mm | |
| Eluents: | A: Water + 0.1 % TFA (Agilent 7) | |
| | B: Acetonitrile + 0.1 % TFA | |
| | | |
| Gradient | 00:00 min | 05%B |
| | 07:00 | 95%B |
| | 07:10 | 100%B |
| | 08:80 | 100%B |
| | 09:00 | 05%B |
| | 12:00 | 05%B |
| | | |
| Flow: | | 2 ml/min |

### Example 8: Cell-uptake experiments

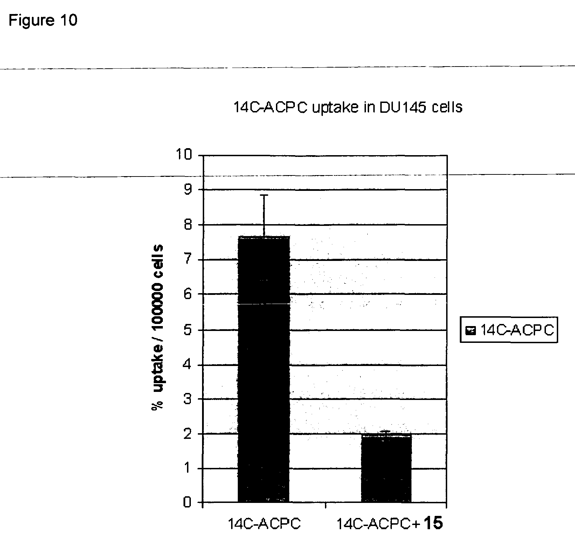

To assess the difference between heterocyclic amino acids and other non-natural amino acids, we studied the uptake of the cyclic amino acid [14C] ACPC into DU145 prostate cancer cells in the presence of excess amount of an heterocyclic amino acid (compound 15). 25000 DU145 cells were seeded per cavity of a 48 well incubation plate (Becton Dickinson; Cat. 353078) and incubated for 2 days in D-MEM / F-12 (1:1) with GlutaMAx (Invitrogen; Cat. 31331) medium supplemented with 10% FCS in an incubator (37°C, 5%CO2). Cells were washed once with PBS and then incubated for 60 minutes at 37°C in uptake buffer (PBS supplemented with 0.1% BSA and containing 5 µM 14C-ACPC ((American Radiolabeled Chemicals Inc.; Cat ARC 0514; Lot. 090220). After incubation, the cells were washed twice with cold PBS, lysed with 1M NaOH, and finally lysates were measured in a scintillation counter in the presence of scintillation fluid.
Surprisingly, the heterocyclic amino acid compound 15 was able to block [14C] ACPC uptake demonstrating high affinity of heterocyclic amino acids towards cellular transporters mediating [14C] ACPC uptake (Figure 10).

### Example 9: PET/CT-imaging in DU145-tumor bearing mice with tracer 45

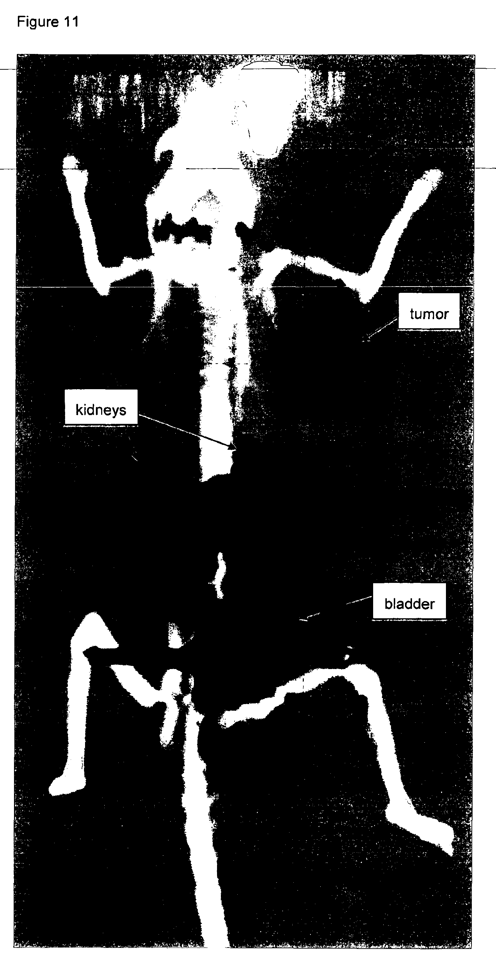

Example 9 was imaged on a microPET/ CT (Inveon, Siemens) in DU145 tumor-bearing mice 50-70min after injection of - 10 MBq radiotracer **45**. High tumor-contrast was visible in DU145 xenografts. Due to rapid renal clearance of this PSMA ligand very low background activity was observed except for kidney and bladder uptake (Reference to Figure 11).

### Example 10: PET/CT-imaging in DU145-tumor bearing mice with tracer 46

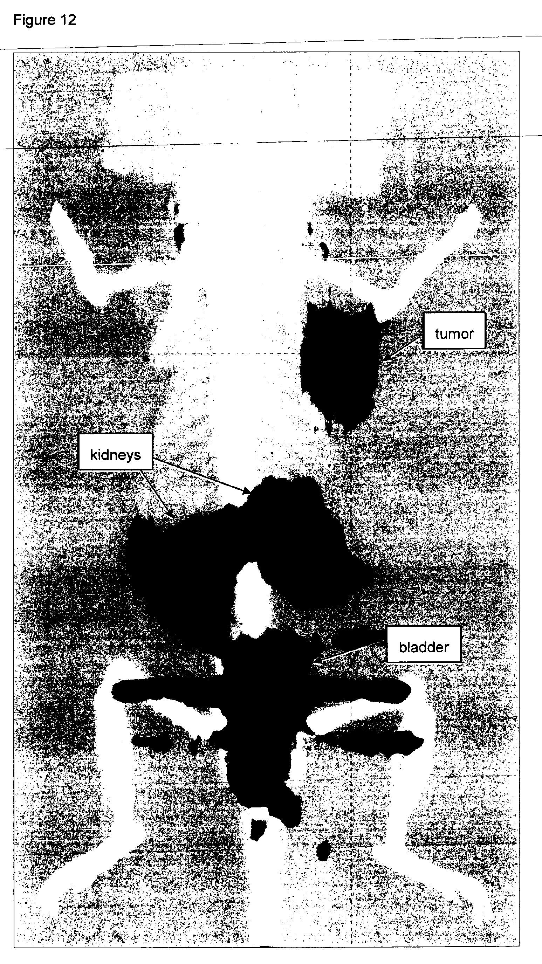

Example 10 was imaged on a microPET/ CT (Inveon, Siemens) in DU145 tumor-bearing mice 50-70min after injection of - 10 MBq radiotracer **46**. High tumor-contrast was visible in DU145 xenografts. Due to rapid renal clearance of this PSMA ligand very low background activity was observed except for kidney and bladder uptake (Reference to Figure 12).

## Claims

1. A compound of formula (I) 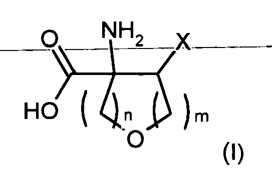 wherein X is Fluorine atom (F),
n = 1 or 2 and
m = 1 or 2 with the proviso that n and m are not both 2,
encompassing single isomers, diastereomers and enantiomers, mixtures thereof and pharmaceutically acceptable salts thereof.

2. A compound of the formula (IIa) or (IIb) 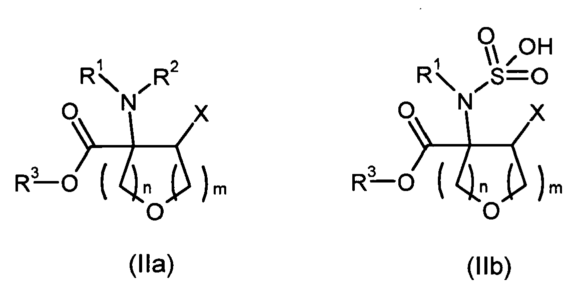 wherein X is Fluorine atom (F),
n = 1 or 2,
m = 1 or 2 with the proviso that n and m are not both 2,
R¹ = hydrogen or an N-protecting group,
R² = hydrogen or an N-protecting group and
R³ = hydrogen or an O-protecting group
or the group NR¹R² is a 1,3-dioxo-1,3-dihydro-2H-isoindol-2-yl (phthalimido) or an azido group;
with the proviso, that at least one of the substituents R¹, R² or R³ is not Hydrogen, encompassing single isomers, diastereomers, enantiomers, mixtures thereof and pharmaceutically acceptable salts thereof.

3. The compound of formula (I) according to claim 1 and compound of formula (IIa) and (IIb) according to claim 2 wherein Fluorine atom (F) is an ¹⁸F or ¹⁹F isotope.

4. The compound of formula (IIa), and (IIb) according to claims 2 to 3 respectively wherein independently from each other N-protecting group is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn), p-Methoxybenzyl (PMB), 3,4-Dimethoxybenzyl (DMPM), Triphenylmethyl, p-Methoxyphenyl (PMP) and the moiety NR¹R² that is a 1,3-dioxo-1,3-dihydro-2H-isoindol-2-yl (phthalimido) or an azido group and O-protecting group is selected from the group of optionally substituted C₁-C₁₀-alkyl, optionally substituted allyl, and optionally substituted C₇-C₁₀-arylalkyl.

5. A compound of the formula (IIIa) or (IIIb) 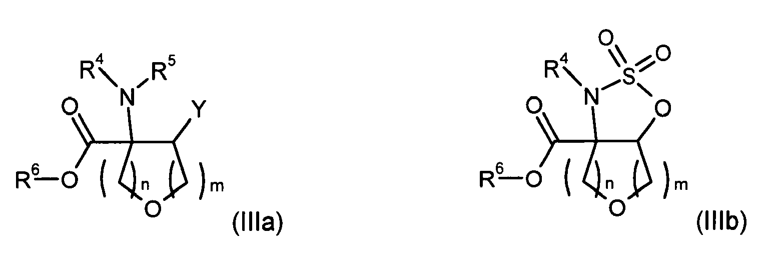 wherein Y is Leaving Group (LG),
n = 1 or 2,
m = 1 or 2 with the proviso that n and m are not both 2,
R⁴ = hydrogen or an N-protecting group,
R⁵ = N-protecting group and
R⁶ = O-protecting group
or the group NR⁴R⁵ is a 1,3-dioxo-1,3-dihydro-2H-isoindol-2-yl (phthalimido) or an azido group,
encompassing single isomers, diastereomers, enantiomers, mixtures thereof pharmaceutically acceptable salts thereof.

6. The compound of formula (IIIa) or (IIIb) according to claim 5 wherein the Leaving Group (LG) is sulphonate or halide.

7. The compound of formula (IIIa) and (IIIb) according to claims 5 to 6 respectively wherein independently from each other N-protecting group is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Benzyl (Bn), p-Methoxybenzyl (PMB), 3,4-Dimethoxybenzyl (DMPM), Triphenylmethyl, p-Methoxyphenyl (PMP) and the moiety NR⁴R⁵ that is a 1,3-dioxo-1,3-dihydro-2H-isoindol-2-yl (phthalimido) or an azido group and O-protecting group is selected from the group of optionally substituted C₁-C₁₀-alkyl, optionally substituted allyl, and optionally substituted C₇-C₁₀-arylalkyl.

8. A composition comprising compounds of the formula (I), (IIa), (IIb), (IIIa), and/or (IIIb) or mixtures thereof and pharmaceutical acceptable carrier or diluent.

9. A direct labelling method for obtaining compound of formula (I), (IIa) and/or (IIb) or mixture thereof.

10. A compound of formula (I), (IIa) or (IIb) for imaging proliferative diseases in mammal.

11. A kit comprising a sealed vial containing a predetermined quantity of a compound having chemical Formula (I), (IIa), (IIb), (IIIa) or (IIIb) and pharmaceutically acceptable salts of inorganic or organic acids thereof, hydrates, complexes, esters, amides, and solvates thereof.

12. A method for monitoring tumor and/or metastases size by PET imaging a patient using compound of Formula (I), (IIa) or (IIb) or mixture thereof.

13. The compound accordingly to claims 1 to 7 respectively (3*SR*,4*SR*)-3-Amino-4-[¹⁸F]fluorotetrahydrofuran-3-carboxylic acid 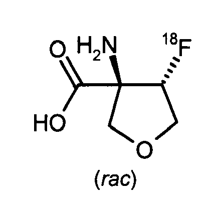 (3*SR*,4*SR*)-3-Amino-4-fluorotetrahydrofuran-3-carboxylic acid 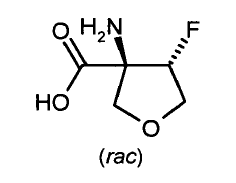 (3*R*,4*R*)-3-Amino-4-[¹⁸F]fluorotetrahydrofuran-3-carboxylic acid 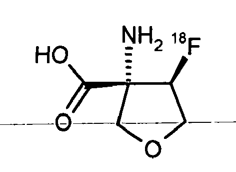 (3*R*,4*R*)-3-Amino-4-fluorotetrahydrofuran-3-carboxylic acid 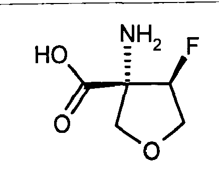 (3*RS*,4*RS*)-4-Amino-3-[¹⁸F]fluoro-3,4,5,6-tetrahydro-2H-pyran-4-carboxylic acid 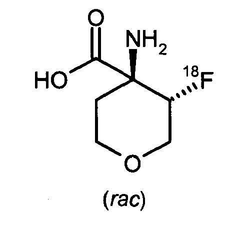 *N-*(*tert-*Butoxycarbonyl)-*N-*[(3*SR*,4*SR*)-3-(*tert-*butoxycarbonyl)-4-fluorotetrahydrofuran -3-yl]sulfamic acid 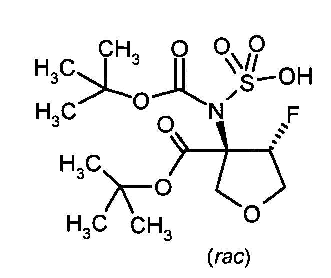 *N-*(*tert-*Butoxycarbonyl)-*N-*[(3*SR*,4*SR*)-3-(*tert-*butoxycarbonyl)-4-[¹⁸F]fluorotetrahydrofuran -3-yl]sulfamic acid 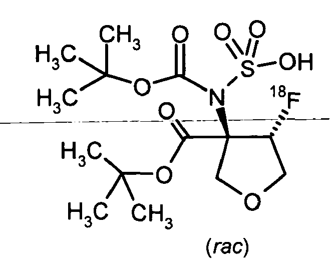 *N-*(*tert-*Butoxycarbonyl)-*N-*[(3*R*,4*R*)-3-(*tert-*butoxycarbonyl)-4-[¹⁸F]fluorotetrahydrofuran -3-yl]sulfamic acid 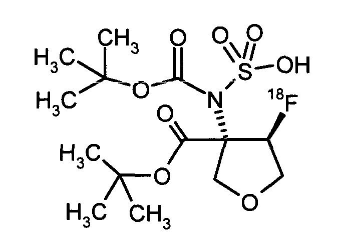 *N-*(*tert-*Butoxycarbonyl)-*N-*[(3*SR*,4*SR*)-4-(*tert-*butoxycarbonyl)-3-[¹⁸F]fluoro-3,4,5,6-tetrahydro -2*H-*pyran-4-yl]sulfamic acid 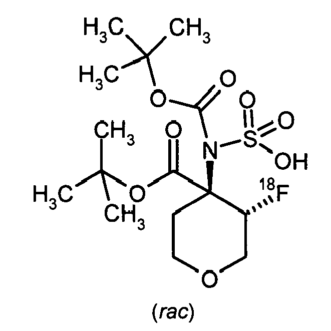 *tert-*Butyl (3*SR*,4*SR*)-3-[(*tert-*butoxycarbonyl)amino]-4-{[(trifluoromethyl)sulfonyl]oxy}-tetrahydrofuran-3-carboxylate 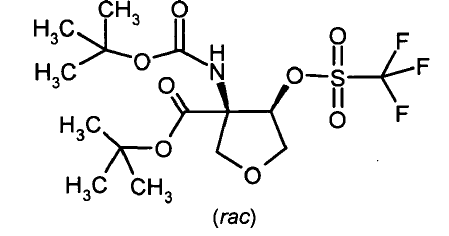 *tert-*Butyl (3*RS*,4*RS*)-3-[(*tert-*butoxycarbonyl)amino]-4-(mesyloxy)tetrahydrofuran-3-carboxylate 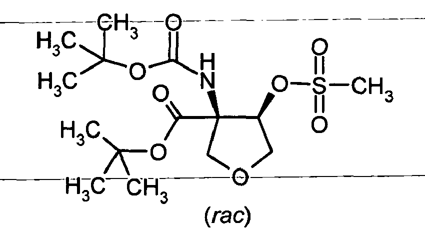 *tert-*Butyl (3*RS*,4*RS*)-3-[(*tert-*butoxycarbonyl)amino]-4-(tosyloxy)tetrahydrofuran-3-carboxylate 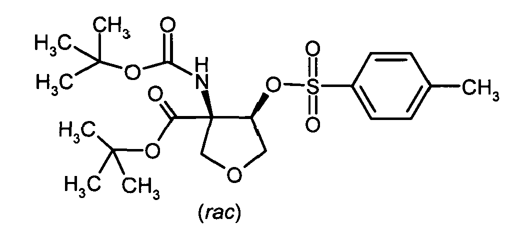 Methyl (3*RS*,4*RS*)-3-[(*tert-*butoxycarbonyl)amino]-4-(mesyloxy)tetrahydrofuran-3-carboxylate 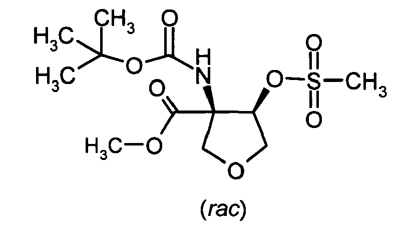 Methyl (3*RS*,4*RS*)-3-[(*tert-*butoxycarbonyl)amino]-4-(tosyloxy)tetrahydrofuran-3-carboxylate 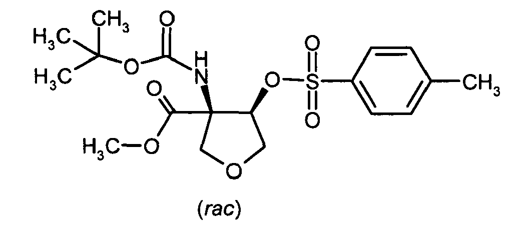 Di-*tert-*butyl (3a*RS*,6a*RS*)-3a,4,6,6a-tetrahydro-3*H-*furo[3,4*-d*]-1,2,3-oxathiazole-3,3a-dicarboxylate 2,2-dioxide 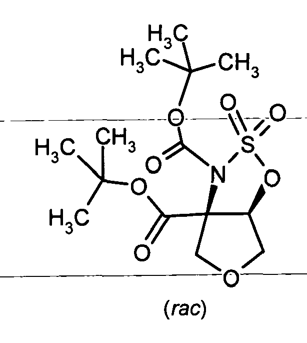 Di-*tert-*butyl (3a*S*,6a*S*)-3a,4,6,6a-tetrahydro-3*H-*furo[3,4-*d*]-1,2,3-oxathiazole-3,3a-dicarboxylate 2,2-dioxide 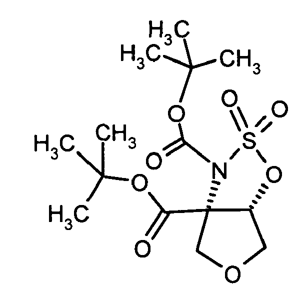 Di-tert butyl (3a*RS*,7a*RS*)-3,3a,4,5,7,7a-hexahydropyrano[4,3*-d*]-1,2,3-oxathiazole-3,3a-dicarboxylate 2,2-dioxide 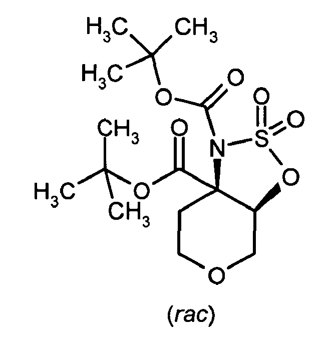 *tert-*Butyl (3*RS*,4*RS*)-4-[(*tert-*butoxycarbonyl)amino]-3-(mesyloxy)-3,4,5,6-tetrahydro-2H-pyran-4-carboxylate 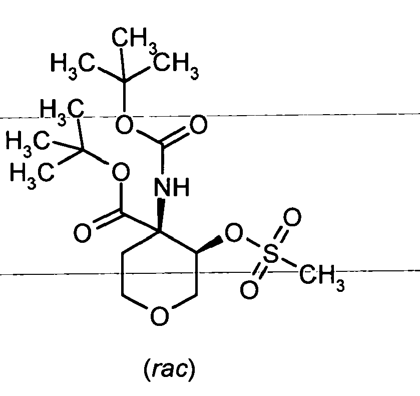 and
*tert-*Butyl (3*RS*,4*RS*)-4-[(*tert-*butoxycarbonyl)amino]-3-(tosyloxy)-3,4,5,6-tetrahydro-2H-pyran-4-carboxylate
